# EUROPEAN PATENT SPECIFICATION

(11) **EP 1 694 342 B1**
(45) Date of publication and mention of the grant of the patent: **04.11.2020**
(21) Application number: 04810817.9
(22) Date of filing: 12.11.2004
(51) Int. Cl.: A61K 35/14, A61K 39/02, A61K 38/00, A61P 31/00

(54) **METHODS OF USING GELSOLIN TO TREAT OR PREVENT BACTERIAL SEPSIS**
VERFAHREN ZUR VERWENDUNG VON GELSOLIN ZUR BEHANDLUNG ODER PRÄVENTION VON BAKTERIELLER SEPSIS
PROCEDES D'UTILISATION DE LA GELSOLINE POUR TRAITER OU PREVENIR LA SEPSIE BACTERIENNE

(30) Priority: 12.11.2003 US 519286 P
(43) Date of publication of application: 30.08.2006
(73) Proprietor: TRUSTEES OF THE UNIVERSITY OF PENNSYLVANIA, Philadelphia, PA 19104-3147 (US)
(72) Inventor: BUCKI, Robert, Philadelphia, PA 19102 (US); CHABY, Richard, 92220 Bagneux (FR); JANMEY, Paul, A., Media, PA 19063 (US); STOSSEL, Thomas, P. Hematology Division, Karp 6 Boston, MA 02115 (US)
(74) Representative: Gill, Siân Victoria
(86) International application number: PCT/US2004/037763
(87) International publication number: WO 2005/046454

(56) References cited:
- WO-A1-00/55350
- WO-A1-91/15770
- WO-A1-95/09645
- WO-A1-98/24465
- WO-A2-2004/023973
- ROTHENBACH PATRICIA A ET AL: "Recombinant plasma gelsolin infusion attenuates burn-induced pulmonary microvascular dysfunction" JOURNAL OF APPLIED PHYSIOLOGY, AMERICAN PHYSIOLOGICAL SOCIETY, US LNKD- DOI:10.1152/JAPPLPHYSIOL.01074.2002, vol. 96, no. 1, 2 May 2003 (2003-05-02), pages 25-31, XP002529488 ISSN: 8750-7587 [retrieved on 2003-05-02]
- SUHLER E ET AL: "Decreased plasma gelsolin concentrations in acute liver failure, myocardial infarction, septic shock, and myonecrosis" CRITICAL CARE MEDICINE 199704 US, vol. 25, no. 4, April 1997 (1997-04), pages 594-598, XP008122382 ISSN: 0090-3493
- BUCKI ROBERT ET AL: "Inactivation of endotoxin by human plasma gelsolin" BIOCHEMISTRY, vol. 44, no. 28, July 2005 (2005-07), pages 9590-9597, XP002582953 ISSN: 0006-2960
- BUCKI R ET AL: "Bacterial endotoxin as inhibitor of the enzymatic activity of human thrombin" EUROPEAN JOURNAL OF HAEMATOLOGY 200606 GB LNKD- DOI:10.1111/J.0902-4441.2005.T01-1-EJH2448 .X, vol. 76, no. 6, June 2006 (2006-06), pages 510-515, XP002582954
- BUCKI R ET AL: "Extracellular gelsolin binds lipoteichoic acid and modulates cellular response to proinflammatory bacterial wall components" JOURNAL OF IMMUNOLOGY 20081001 AMERICAN ASSOCIATION OF IMMUNOLOGISTS USA, vol. 181, no. 7, 1 October 2008 (2008-10-01), pages 4936-4944, XP002583984
- MOUNZER K C ET AL: "Relationship of admission plasma gelsolin levels to clinical outcomes in patients after major trauma" AMERICAN JOURNAL OF RESPIRATORY AND CRITICAL CARE MEDICINE 1999 US, vol. 160, no. 5 I, 1999, pages 1673-1681, XP002582956 ISSN: 1073-449X
- BUCKI R ET AL: "Antibacterial Activities of Rhodamine B-Conjugated Gelsolin-Derived Peptides Compared to Those of the Antimicrobial Peptides Cathelicidin LL37, Magainin II, and Melittin" ANTIMICROBIAL AGENTS AND CHEMOTHERAPY 200405 US LNKD- DOI:10.1128/AAC.48.5.1526-1533.2004, vol. 48, no. 5, May 2004 (2004-05), pages 1526-1533, XP002582957 ISSN: 0066-4804
- LEE P-S ET AL: "Plasma gelsolin is a marker and therapeutic agent in animal sepsis", CRITICAL CARE MEDICINE, WILLIAMS AND WILKINGS COMPANY, BALTIMORE, MA, US, vol. 35, no. 3, 1 March 2007 (2007-03-01), pages 849-855, XP008122402, ISSN: 0090-3493, DOI: DOI:10.1097/01.CCM.0000253815.26311.24

### SEQUENCE LISTING

<110> The Trustees of the University of Pennsylvania
   Bucki, Robert
   Chaby, Richard
   Janmey, Paul A.
   Stossel, Thomas P.
<120> Methods of Using Gelsolin To Treat Or Prevent Bacterial Sepsis
<130> 22253-77008
<140> TO Be Assigned
   <141> 2004-11-12
<150> 60/519,286
   <151> 2003-11-12
<160> 1
<170> PatentIn version 3.3
<210> 1
   <211> 10
   <212> PRT
   <213> Artificial
<220>
   <223> peptide fragment of gelsolin
<400> 1
   Gln Arg Leu Phe Gln Val Lys Gly Arg Arg
   1               5                   10

## Description

### FIELD OF THE INVENTION

The present invention relates to treating, preventing or neutralizing septic shock in mammals.

### BACKGROUND OF THE INVENTION

Septic shock from bacterial endotoxins, triggered by release of lipopolysaccharide (LPS) molecules from the outer wall of gram-negative bacteria, is a major cause of human death for which there has previously been no effective treatment once the complex inflammatory pathways have been activated. Delivery of LPS from external fluids to the cell membrane, and ultimately to the LPS receptors is complex, involving a number of external proteins and other factors (Erridge et al., Microbes Infect. 4:837-851 (2002); Bosshart et al., FEES Lett. 553:135-140 (2003); Thomas et al., Biochem. Biophys. Res. Commun. 307:133-138 (2003); Rustici et al., (1993) Science 259, 361-365 (1993)). A critical factor in LPS toxicity is the aggregation-state of this amphipathic molecule, *e.g.*, when LPS are packed into lamellar phases toxicity is low, but high when present in non-lamellar phases (Brandenburg et al. Carbohydr. Res. 338:2477-2489 (2003)). As a result, strategies to counteract LPS in serum or cerebrospinal fluid have involved several types of small molecules and proteins, including anti-LPS antibodies, such as transferrin, alpha 2-macroglobulin, Gc-globulin (Berger et al., Clin. Chim. Acta 163:289-299 (1987); Berer et al., Eur. J. Clin. Invest. 20:66-71 (1990)) and MD-2 (Shimazu et al., J. Exp. Med. 189:1777-1782 (1999)).

Lipoprotein particles, such as LPS-binding protein (LBP) are also ligands for LPS (Wright et al., J. Exp. Med. 170:1231-1241 (1989)), in which case a rise in plasma lipid levels during sepsis may be a protective response since LPS bound to other molecules appears to lose its stimulatory effect on cells (Casas et al., J. Surg. Res. 59:544-552 (1995); Bannerman et al., J. Daily Sci. 86:3128-3137 (2003)), and promotes cytokine tolerance in hepatocytes (Harris et al., J. Endotoxin Res. 9:45-50 (2003)). Both LBP and CD14 promote partitioning of LPS into lipoprotein complexes (Vreugdenhil et al., J. Immunol. 170:1399-1405 (2003); Wurfel et al., J. Exp. Med. 181:1743-1754 (1995)), and MD. More importantly, low concentrations of LBP have been shown to enhance the LPS-induced activation of mononuclear cells, whereas increased LBP concentrations inhibit LPS-induced cell stimulation (Gutsmann et al., Infect. Immun. 69:6942-6950 (2001)). LBP also delivers LPS to its receptors at the cell membrane. This dual effect of the protein on LPS function reinforces the importance of the lipid aggregation-state, and confirms that toxicity does not result from mass concentration alone. Related to this effect may be the finding that low doses of antimicrobial agents can lead to exacerbation of the toxicity associated with LPS (Tauber et al., J. Infect. Dis. 156:456-462 (1987); Mertsola et al., Pediatr. Infect. Dis. J. 8:904-906 (1986)).

Gelsolin is a normal serum protein, but unlike other mammalian proteins, gelsolin has both cytoplasmic and secreted isoforms derived by alternative slicing of the message from a single gene (Sun et al., J. Biol. Chem. 274:33179-33182 (1999)). Although originally identified as a cellular protein, cytoplasmic gelsolin also exists as an abundant secreted isoform of nearly identical structure and is a product of the same gene (Kwiatkowski et al., Nature 323:455-458 (1986)). It has a six-fold sequence repeat structure that is highly conserved among gelsolins of vertebrate species, and that is characteristic of a large family of gelsolin-related proteins (Kwiatkowski, Curr. Opin. Cell Biol. 11: 103-108 (1999)).

Secretory gelsolin, now called "plasma gelsolin," circulates in human and rodent blood at concentrations of 250 ±50 µg/L (Lind et al., Am. Rev. Respir. Dis. 138:429-434 (1988)). Plasma gelsolin has a processed signal sequence, but is otherwise identical to the cellular form, with the exception of a 23 amino acid stretch at the amino terminus of the molecule, designated the "plasma extension." Therefore, the plasma form of gelsolin is slightly larger (84 kDa), than the cellular variant (82kDa). Recombinant human gelsolin (rhGSN) (Biogen IDEC, Inc., Cambridge, MA) is produced in *E. coli,* and though it has the same primary structure as native protein, under standard conditions of purification, it differs from natural human plasma gelsolin by a disulfide bond that is present in the natural protein. The recombinant protein is, therefore, properly oxidized after purification, and its structure and functions are indistinguishable from purified human plasma gelsolin (see, Wen et al., Biochemistry 35:9700-9709 (1996)).

Although secreted by many tissues, the major source of human plasma gelsolin is straited muscle (Kwiatkowski et al., J. Biol. Chem. 263:8239-8243 (1988)). This form of gelsolin appears to be distributed throughout extracellular fluids and has a typical residence time in plasma (Lind et al., J. Clin. Invest. 78:736-742 (1986)). It is not modified post-translationally by glycosylation or other reactions, nor is it an acute phase reactant. In fact, little information exists concerning its function or regulation of its production. However, it is known that levels of gelsolin drop in clinical settings when inflammation or sepsis occurs, *i.e.,* tissue injury associated with breakdown of membrane barriers and exposure of actin to the extracellular environment results in reductions in plasma gelsolin levels (Lee et al., N. Engl J. Med 326:1335-1341 (1992)).

As a result, plasma gelsolin depletion precedes and predicts complications of severe injury, such as respiratory failure and death in animals including humans (Mounzer et al., Am J. Respir. Crit. Care 111ed. 160:1673-1681 (1999); DiNubile et al., Blood 100:4367-4371 (2002)). For example, a drop in plasma gelsolin levels to <50% normally is a strong predictor of adverse clinical outcomes associated with massive inflammation, although neither a causative role, nor a treatment has been identified based on this finding (Dahl et al., Shock 12:102-104 (1999); Dahl et al., Crit. Care Aled; 31:152-156 (2003); *Lind et al.,* 1988, *20 supra;* Smith et al., Blood 72:214-218 (1988); Suhler et al., Crit. Care Med. 25:594-598 (1997)). However, replacement of depleted plasma gelsolin levels has attenuated the pathologic sequelae of severe primary injuries in animal models (Christofidou-Solomidou et al., J. Investig. Med. 50: 54-60 (2002); Rothenbach et al., J. Appl. Physiol. 96:25-31 (Jan. 2004)), supporting the findings of the present invention.

In response to acute trauma and/or infection, abundant but normally intracellular G-actin (monomeric actin) is released into extracellular spaces from damaged or dying cells and tissues and circulates in fluid and blood. Once released, G-actin has a strong tendency to polymerize to F-actin. The persistance of filaments of F-actin in the microvasculature of mammals can result in venous obstruction, pulmonary microthrombii and/or endothelial injury, and induces or enhances platelet agglutination in the blood, thereby triggering thrombus development (Scarborough et al., Biochem. Biophys. Res. Commun. 100:1314-1319 (1981)). Combined, these effects alter the characteristics of normal vascular flow in mammals, and in turn, can result in actin toxicity disorders and contribute to the pathogenesis of organ injury at sites removed from the primary insult (Dahl et al., Crit. Care Med. 26:285-289 (1998); Lee *et al.,* 1992, *supra;* Mounzer *et al.,* 1999, *supra,* see also US Patent No. 5,593,964).

However, various actin-regulating proteins contribute to the reversible conversion of filaments ("gel") and monomers (liquid "sol"), and changes occur depending on extracellular stimuli (see, US Patent No. 6,271,353). Plasma gelsolin and secreted Gc-globulin act in a coordinated manner, representing an "actin-scavenger system," to depolymerize and remove the actin filaments released from damaged cells. Gelsolin binds to both monomeric and filamentous actin (Yin et al., Nature 281:583-586 (1979)), although following injury, gelsolin preferably binds to and severs the actin filaments to promote rapid depolymerization (Sun *et al,* 1999, *supra),* whereas Gc-globulin binds to the actin monomers to shift the actin monomer/polymer equilibrium back toward depolymerizations and prevent repolymerization (Goldschmidt-Clermont et al., J. Clin. Invest. 81:1519-1527 (1988)).

This binding requires the presence of micromolar concentrations of calcium (Ca²⁺), which may include added calcium or endogenously available Ca²⁺ in the patient. Moreover the binding is very tight, having a dissociation constant in the nanomolar range. In fact, when gelsolin binds actin filaments in the presence of calcium, it ruptures or severs the filaments at the binding site by breaking the noncovalent bonds holding actin monomers together within the polymer (Janmey et al., Biochemistry 24:3714-3723 (1985)). Following the actin severing reaction, gelsolin remains tightly bound to one end of the polarized actin filament, the end conventionally defined as "barbed." This is also the end that rapidly exchanges monomers.

Removing calcium by chelation does not dissociate gelsolin from the barbed ends of the actin filaments. Instead, phosphoinositides (also referred to as phosphorylated inositol phospholipids, or "PPIs"), which are important signal transduction intermediates, effect this separation at the plasma membrane. Gelsolin binds with high affinity and selectivity to PPI and to lysophosphatidic acid (LPA) (Janmey et al., J. Biol. Chem. 262:12228-12236 (1987); Meerschaert et al., Embo J. 17:5923-32 (1998)). PPIs, therefore, regulate the intracellular actin-binding function of gelsolin, leading to the hypothesis that a reciprocal relationship, between calcium transients and membrane phosphoinositide synthesis and degradation, regulates gelsolin and cellular actin remodeling responses (Stossel, J. Biol. Chem. 264, 18261-18264 (1989)). Gelsolin binding to LPA, modulates its receptor-mediated biological effects, leading to the belief that it may act as a carrier of LPA to some cellular receptors, and buffers bioactive inflammatory lipid mediators (Goetzl et al., J. Biol. Chem. 275:14573-14578 (2000).

Calcium and phosphoinositides also control the actin-binding functions of plasma gelsolin *in vitro* (Janmey et al., Chem. Biol. 5:R81-85 (1998)). The PPI regulatory site of gelsolin resides within a 20 residue linear sequence that connects the first and second folded domains of the protein. Biochemical and mutational studies have implicated 10 strategically organized basic and hydrophobic amino acids (160-169, SEQID No:1 QRLFQVKGRR) in the 684-residue plasma gelsolin molecule that accommodate tight binding to the negatively-charged phosphomonoesters and hydrophobic acyl chains of anionic phospholipids (Janmey et al., J. Biol Chem 267:11818-11823 (1992). Synthetic peptides of this sequence have a PPI binding affinity similar to that of intact gelsolin *(Id).*

The methods used to measure the function of gelsolin, have exploited gelsolin's calcium-dependent actin filament severing function, or actin monomer nucleation activities, or the actual mass of gelsolin, using western blotting or immunochemical (usually ELISA) assays. The assays used to quantify gelsolin have relative advantages and disadvantages. For example, the assays measure only severing activity that is directly measurable fluorimetrically (Janmey et al., Blood 70:524-530 (1987)) and the assays are specific for free gelsolin, but actin and lipid binding to gelsolin inhibit this function. Nucleation activity and the structural assays do not differentiate free from complexed gelsolin. However, different assays have thus far yielded very similar results for plasma gelsolin levels in control samples, and generally consistent degrees of diminution in plasmas from injured animals or humans, with the exception that severing activity is lower than expected. However, there are also unexplained data from studies showing diminished severing, but not total gelsolin activity in serum samples with no evidence of actin, suggesting that another ligand may have bound the gelsolin.

While there is no apparent relation between the function or metabolism of LPS and either LPA or PIP2 (phosphatidylinositol-4,5-bisphosphate), these lipids share two unusual and essential structural characteristics, and as acidic lipids, they superficially resemble LPS in that all have phosphomonoesters juxtaposed to a hydrophobic interface (Erridge *et al.,* 2002, *supra).* The active site of each molecule contains a phosphomonoester emanating from a carbohydrate base, glycerol in the case of LPA, and a sugar ring for LPS and PIP2. These moieties, which are present in few other lipids, present possibilities for electrostatic and hydrogen-binding interactions that stabilize both lipid-protein and lipid-lipid complexes ((Liepina et al., Biopolymers 71:49-70 (2003)). Also, none of these lipids forms lamellar phases by itself *(e.g.,* Flanagan et al., Biophys. J. 73:1440-1447 (1997)), LPA and PIP2 binding to gelsolin appears to be strongest when the lipid is in micelles or putative lipid clusters within bilayer vesicles (Meerschaert et al., Embo J. 17:5923-5932 (1998); Tuominen et al., Eur. J. Biochem. 263:85-92 (1999); Janmey et al., J. Biol. Chem. 262:12228-12236 (1987)).

Plasma gelsolin depletion contributes to the pathophysiology of pulmonary microvascular dysfunction resulting from a primary bum- or bacterially-induced inflammation, and inflammation-induced lung injury (Rothenbach *et al.,* 2004, *supra).* A blatant clinical example of secondary tissue injury associated with, and resulting from, a depletion of gelsolin, is adult respiratory distress - multiple organ dysfunction syndrome (ARDS/MODS) (Ware et al., N. Engl. J. Med. 342:1334 (2000)). Plasma gelsolin levels have dropped to reportedly, on average, 30% of normal values in established ARDS cases (Yin *et al,* 1979, *supra).* An unimpressive track record of pharmacologic interventions in established ARDS, suggests that once full-blown ARDS occurs, it may no longer be possible to inhibit the broad spectrum of activated inflammatory mediators that are involved.

WO 91/15770 discloses a method for treating actin-related disorders by decreasing the concentration of free actin in the plasma of an animal by administering native and modified actin-binding proteins, such as actin-binding regions of gelsolin.

Accordingly, at the present time, there is no agent to clinically neutralize endotoxins, or prevent or avert the resulting bacterial sepsis, and often death. A need has remained, therefore, until the present invention, to better understand PPI-binding proteins and the lipid ligands of these proteins, how they affect the function of gelsolin, how altering PPI levels in the body works to reorganize the actin cytoskeleton, and the role of these lipids and lipid binding proteins, such as gelsolin, in inflammation, particularly with regard to developing methods for treating, alleviating, or even preventing, the onset and pathology of bacterially-caused endotoxemia, sepsis, inflammation-induced pulmonary microvascular dysfunction following severe burns or myocardial infarction, ARDS or cystic fibrosis.

### SUMMARY OF THE INVENTION

Gelsolin-depletion contributes to the pathophysiology of microvascular dysfunction during inflammation in trauma and burn patients, as well as, in septic shock resulting from bacterial endotoxins, triggered by release of lipopolysaccharide (LPS) molecules from infecting gram-negative bacteria. As a result, the present disclosure provides methods to counteract the decreasing plasma gelsolin levels at a sufficiently early time point in inflammation-induced injury by the infusion of gelsolin, that microvascular dysfunction is averted. In fact, in accordance with preferred aspects of the disclosure, intravenous infusion of synthetic, recombinant human gelsolin (recombinant replacement therapy) completely prevented, the irreversible tissue damage and increased microvascular permeability otherwise associated with bacterial endotoxin production, and death. Thus, the disclosure provides important new evidence to support the finding that circulating gelsolin has an important protective function against the pathophysiology of systemic inflammation. It is an object of the present invention to provide gelsolin, or a functionally equivalent peptide fragment thereof, for use as a medicament in a method of preventing, neutralizing or reducing endotoxemia or endotoxin-induced septic shock in a patient in vivo, wherein the patient is subject to or susceptible to gram negative bacterial infection. It is a further object of the present invention to provide a pharmaceutical composition comprising gelsolin for use as stated above, said pharmaceutical composition comprising:(1) gelsolin, or functionally equivalent peptide fragment thereof, wherein such gelsolin or said fragment is substantially free of natural contaminants; and (2) a pharmaceutically acceptable vehicle; and (3) a sufficient amount of Ca2+ to activate the binding capability of exogenously administered gelsolin, for use in a method of preventing, neutralizing or reducing endotoxemia or endotoxin-induced septic shock in a patient.It is a further object of the present invention to provide a use of gelsolin, or functionally equivalent peptide fragment thereof, in the manufacture of a medicament for preventing, neutralizing or reducing endotoxemia or endotoxin-induced septic shock in a patient. It is a further object of the present invention to provide gelsolin for use in a method of predicting clinical outcome associated with massive inflammation in a patient susceptible to inflammatory shock or endotoxin-induced sepsis, said method comprising measuring the circulating gelsolin concentration in the patient, wherein a decrease ≤ 30% of normal pre-trauma or pre-infection gelsolin levels predicts such adverse outcome and predicts a need for gelsolin therapy.

It is an object of the present disclosure to provide a method for increasing the concentration of gelsolin or functionally equivalent peptide fragment thereof, in blood or extracellular fluid of a patient *in vitro* or *in vivo,* wherein such increased concentration of gelsolin is needed, said method comprising administering to the patient a therapeutically effective amount of gelsolin, or functionally equivalent peptide fragment thereof.

It is a further object to provide methods for decreasing the concentration of bacterial LPS in blood or extracellular fluid of a patient, thereby preventing, neutralizing or reducing endotoxemia or endotoxin-induced septic shock in a patient *in vitro* or *in vivo,* wherein the patient is subject to or susceptible to gram negative bacterial infection, said method comprising administering a therapeutically effective amount of gelsolin, or functionally equivalent peptide fragment thereof, to decrease the concentration of bacterial LPS and protect the patient from endotoxemia or endotoxin-induced sepsis. Consequently, the method further blocks, reduces or ameliorates bacterial LPS-induced disruption of mammalian cellular responses or formation of toxic structures *in vitro* or *in vivo.* It also blocks, reduces or ameliorates the inhibition of fibrinolysis by excess, extracellular free actin in blood or extracellular fluid of a patient *in vitro* or *in vivo.* In addition it restores or maintains normal

It is an additional object that each embodied method be useful *in vitro* or in the actual gelsolin replacement therapy of a patient.

When such methods are used *in vivo,* it is a further object that the method effectively inhibits, ameliorates or prevents secondary tissue injury in the patient resulting from an accumulation of excess bacterial LPS, said method comprising administering a therapeutically effective amount of gelsolin, or functionally equivalent peptide fragment thereof. Moreover, the methods are effective even when the secondary tissue injury in the patient is remote from the-site of primary infection or trauma.

It is yet another object to provide method for predicting adverse clinical outcome associated with massive inflammation in a patient susceptible to inflammatory shock or endotoxin-induced sepsis, said method comprising measuring the circulating gelsolin concentration in the patient, wherein a decrease ≤ 30% of normal, pre-trauma or pre-infection gelsolin levels predicts such adverse outcome and predicts a need for gelsolin therapy. Also provided are the compositions of gelsolin or active fragment thereof as used in the foregoing methods.

Additional objects, advantages and novel features of the disclosure will be set forth in part in the description, examples and figures which follow, all of which are intended to be for illustrative purposes only. The invention is defined in the claims. Aspects, embodiments and examples of the present disclosure which do not fall within the scope of the claims do not form part of the invention and are provided merely for illustrative purposes.

### BRIEF DESCRIPTION OF THE FIGURES

The foregoing summary, as well as the following detailed description of the invention, will be better understood when read in conjunction with the appended drawings. It should be understood, however, that the invention is not limited to the precise arrangements and instrumentalities shown.

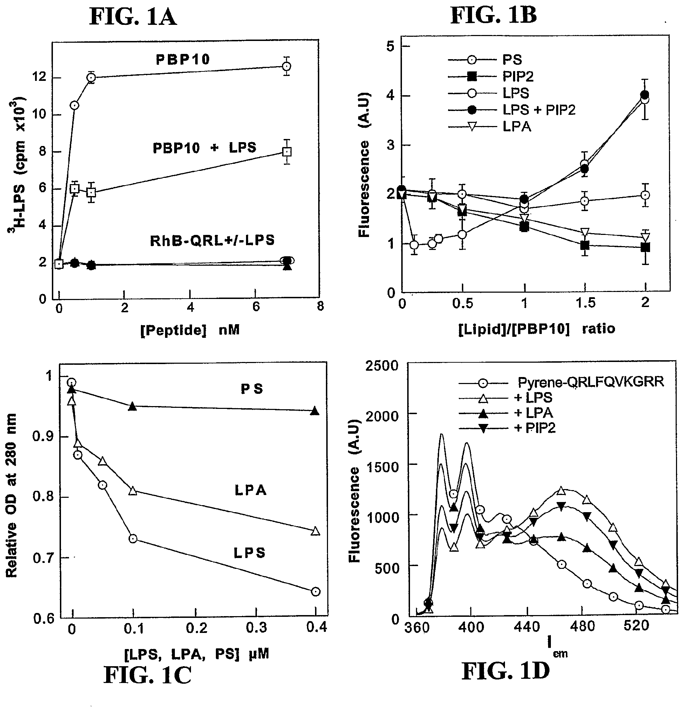

FIGs. 1A-1D graphically depict the interaction of PBP10 and gelsolin with LPS. FIG. 1A shows the binding of ³H-labeled LPS to PBP10 peptides ±cold LPS (PBP10 shown by open circles; PBP10 + LPS shown by open squares; rhodamine-B (RhB)-labeled QRL fragment ±LPS shown by filled circles). FIG. 1B shows the optical density (OD) of gelsolin in solutions containing varying amounts of LPS (open circles) or LPA (open triangles) or PS (filled triangles). FIG. 1C shows rhodamine-B fluorescent changes of PBP10-peptide in the presence of different lipids or controls (PS, open circles; PIP2, closed squares; LPS, open circles; LPS + PIP2, closed circles; LPA, open triangles). FIG. 1D shows the fluorescence emission spectra of pyrene-SEQID No: 1 (open circles) in the presence of LPS from *E.coli* (peptide + LPS; open triangles), PIP2 (peptide + PIP2; filled inverted triangles) and LPA (peptide+ LPA; filled triangles). Data shown in FIGs 1A, 1B and 1C are means ±SD of 2-4 experiments; FIG. 1D data are representative for three independent experiments.

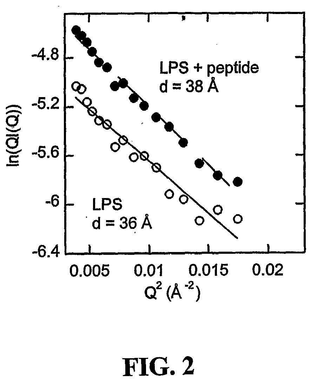

FIG. 2 graphically shows a neutron scattering plot of the molecular structure of LPS + gelsolin SEQID No:1.

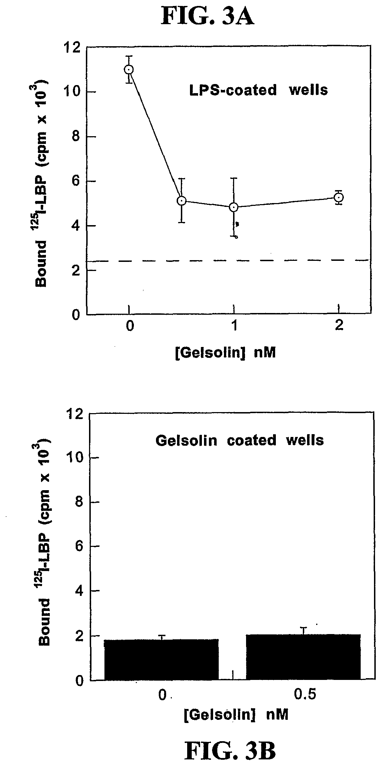

FIGs. 3A and 3B graphically show that gelsolin competes with and inhibits the binding of LPS-binding protein, LBP, from binding to LPS. FIG. 3A shows ¹²⁵I-LBP does not bind to LPS coated wells that had been previously incubated with gelsolin; but gelsolin does not interact directly with the LBP (FIG. 3B). Data shown are mean ±SD of 4 experiments.

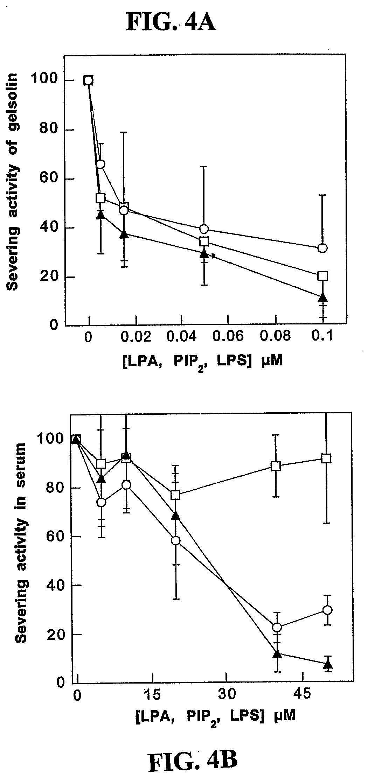

FIGs. 4A and 4B graphically show the effect of LPA (squares), PIP2 (circles) and LPS from *E.coli* (triangles) on actin filament severing activity of recombinant human gelsolin (rhGLS) (FIG. 4A), or human blood plasma (FIG. 4B). Severing activity was determined from the rate of decreased fluorescence during depolmerization following the addition of rhGLS or blood plasma to an F-actin solution polymerized with 50% pyrene labeled G-actin. Data shown are mean ±SD of 4 to 6 experiments.

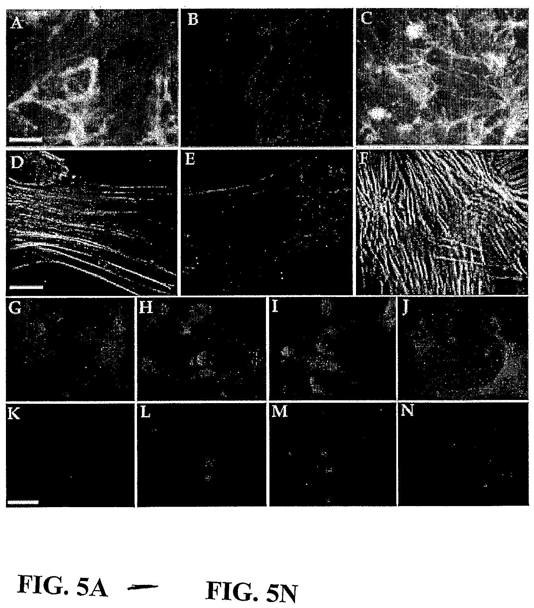

FIGs. 5A-5N photographically depict the LPS-induced disruption of cellular responses is inhibited by pre-incubating LPS with plasma gelsolin. Immunostaining for F-actin with phalloidin (FIGs. 5A-5F) was used to demonstrate the prevention by plasma gelsolin of LPS-induced actin cytoskeleton disassembly. Endothelial cells (FIGs. 5A-5C) and astrocytes (FIGs. 5D-5F) were treated with LPS (FIGs. 5B and 5E), or with a combination of LPS and gelsolin (FIGs. 5C and 5F). Control cells are also shown in FIGs. 5A and 5D. LPS induced NF-kB translocation to the nucleus was blocked by plasma gelsolin in FIGs. 5G-4N), as shown by immunostaining for NF-KB with an anti-NF-KB antibody (FIGs. 5G-5J) and cell nuclei with 4',6-diamidino-4-phenylindole dihydrochloride (FIGs. 5L-5M). In astrocytes, addition of TNF-α (FIGs. 5H and 5L), or 4 µM LPS (FIGs. 5I and 5M) caused NF-κB translocation to the nucleus, as compared with control cells (FIGs. 5G and 5K). Preincubation with gelsolin, however, blocked LPS-induced translocation (FIGs. 5J and 5N).

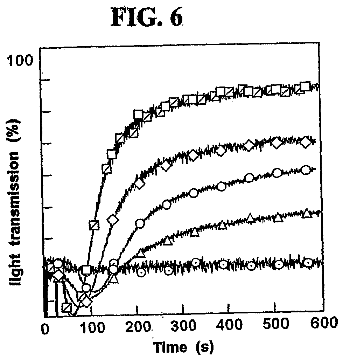

FIGs. 6 graphically depicts the protective effect of gelsolin SEQID No: 1 on LPS-mediated alteration of platelet function, *i.e.,* platelet aggregation after stimulation by collagen, the effect of LPS inhibition, and reversal of the inhibition by gelsolin. Collagen ±25 µM peptide (open squares); LPS + 25 µM peptide+ collagen (open diamonds); LPS + 12 µM peptide+ collagen (open circles); LPS +collagen (open triangles); 25 µM peptide (bottom line of open circles).

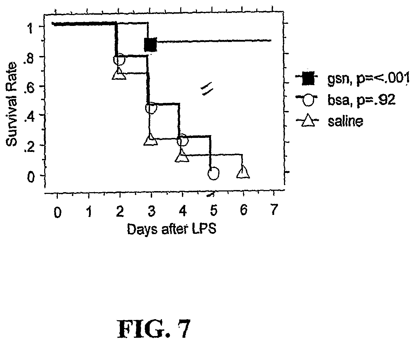

FIG. 7 graphically depicts exogenous plasma gelsolin rescued endotoxemic mice. Mice that received a lethal dose of LPS i.p., were treated with gelsolin (GSN) (filled square), albumin (BSA) (open circles) or saline (open triangles). As shown, all mice that received saline or BSA died within 6 days of LPS treatment; all mice receiving GSN treatment survived and appeared healthy.

### DETAILED DESCRIPTION OF CERTAIN EMBODIMENTS OF THE INVENTION

The present disclosure provides methods for effectively using plasma gelsolin, or active fragments, homologs or analogs thereof, to treat, neutralize, prevent or otherwise control septic shock in a patient, resulting from endotoxins, triggered by release of lipopolysaccharide (LPS) molecules from infecting gram-negative bacteria. As a result, methods are provided to counteract or prevent sepsis, severe inflammation, or other conditions, such as inflammation-induced pulmonary microvascular dysfunction, associated with a marked decrease in plasma gelsolin levels in the patient by modulating LPS. *In vitro* and *in vivo* evidence, including solid phase binding assays, fluorescence, absorbance measurements, and functional assays of actin depolymerizing effects, demonstrate a clear interaction between LPS and gelsolin, and show that gelsolin binds more tightly to LPS than it does to its other known lipid ligands, *e.g*., phosphatidylinositol 4,5 bisphosphate (PIP2) and lysophosphatidic acid (LPA).

The tight and selective binding of gelsolin to LPS has several implications for the function of plasma gelsolin, and the invention, therefore, offers methods to counteract the toxic effects of LPS. Despite a strong clinical correlation between markedly lowered gelsolin levels and susceptibility to inflammatory shock, until the present invention there had been no demonstration or explanation of a therapeutic utility of the interaction between human plasma gelsolin and bacterial LPS that inactivates both the actin scavenging function of gelsolin and the cytotoxic effect of LPS. The current findings indicate that LPS, in addition to LP A and actin, may be the harmful agent responsible for pathologies following trauma, burns or gram negative bacterial infection that leads to gelsolin depletion.

The molecular structure of LPS is heterogeneous, even from a single bacterium, and often is widely variable among different bacteria. Why some forms of LPS are more toxic than others, remains unresolved, but as demonstrated in the examples that follow, not only is the covalent structure of the compound important, but the form of the LPS aggregate appears to be an equally relevant determinant of toxicity. The strong binding of gelsolin to LPS, as well as to LPA and PIP2, points to the importance of aggregate packing in both the biochemistry and biologic function of protein-lipid interactions. LPS, LPA, and PIP2 are similar, but not identical. However, they are, in fact, sufficiently different that highly specific lock and key binding interactions cannot explain these effects.

One of proposed mechanisms of LPS deactivation in blood is LBP-mediated incorporation of LPS molecules into blood lipoproteins (Yu et al., J. Clin. Invest. 99:315-324 (1997). A previous kinetic study suggests, that LPS-binding protein (LBP), a high affinity carrier for LPS, is one of the first plasma components to interact with Lipid A (Tobias et al., Infect. And Immun. 50:73-76 (1985)), thereby influencing interactions of LPS with lipoprotein and cellular targets and serving as an acute phase reactant. It is increased during trauma or infection, which results in increased plasma opsonization capacity (Tobias et al., J Exp. Med. 164:777-793 (1986); Mathison et al., J. Immunol. 149:200-206 (1992)). Therefore, the present findings are significant, showing that gelsolin competes with LBP, further disrupting the onset of sepsis. Accordingly, the present disclosure provides methods for complexing circulating LPS by LPS-binding proteins or lipids, thereby preventing the formation of the toxic structures.

Turning to the Figures, which will be described in greater detail in the Examples that follow, in a solid phase binding assay, ³H-LPS bound in a saturable manner to surfaces that were coated with the PIP2-binding peptide SEQID No:1 (QRLFQVKGRR; also referred to herein as "PBP10"), but not to surfaces coated with a truncated peptide, QRL, which does not bind PIP2. Addition of a substantial (5-fold) excess of unlabeled LPS reduced that binding to PBP10 by approximately 50%, but it had no effect on the small amount of non-specific binding to the control peptide-treated surface. See FIG. 1A. The finding that a small active peptide fragment of gelsolin, such as PBP10, was as effective as whole gelsolin for binding these lipids while, for example, only the correctly folded protein can bind actin, points to a disclosed general binding of LPS through its lipid-binding domain. Under certain conditions, a functionally equivalent peptide fragment may bind better to LPS than does intact gelsolin, perhaps in settings where steric constraints prevent the whole protein from finding the binding site on LPS, while allowing the smaller peptide to do so.

When the term "gelsolin" is used herein, it is herein to mean, whole or intact gelsolin or a biologically active abbreviated sequence forms of gelsolin, and their biologically active analogs (including muteins) having substituted, deleted, elongated, replaced, or otherwise modified sequences which possess LPS-binding bioactivity that is substantially similar to that of native gelsolin, as well as biologically active ammo acid sequences substantially similar to gelsolin. As used herein, "biologically active equivalent peptide analogs" refers to "functionally equivalent peptide fragments," including homologs of gelsolin, preferably SEQID No:1, that contain conservative amino acid substitutions, provided that the peptide analogs which contain the conservative substitutions bind to a PPI (phosphoinositide) regulating the intracellular actin binding function of gelsolin, and have transport-mediating activity, as determined, for example, in an *in vitro* screening assay. As used herein, "conservative amino acid substitution" refers to an amino acid substitution, which does not alter the relative charge or size characteristics of the peptide in which the amino acid substitution is made. Moreover, as used herein, the term "gelsolin" is intended to include naturally occurring or recombinant or synthetically produced isolated gelsolin or functionally equivalent peptide fragments thereof. All are included within the present invention.

Binding of LPS to intact gelsolin was evident by a change in UV absorbance (FIG. 1B) of at least 10%, more preferably 20%, more preferably 30%, with a maximal decrease seen of -35%, although it could be greater. Such a decrease in tyrosine and tryptophan fluorescence, due to decreased absorbance, has been documented as an assay for PIP2 binding to gelsolin (Lind *et al.,* 1997, *supra).* LPA also diminished gelsolin's absorbance, but PS had no effect. Moreover, binding of PPIs and LPA to gelsolin strongly inhibited gelsolin's actin filament severing function.

The fluorescence of rhodamine-B-labeled-SEQID NO:1 (QRLFQVKGRR) is also changed by LPS, as shown in FIG. 1C. LPS and PIP2, but not phosphatidylserine (PS), induced identical concentration-dependent fluorescence quenching of PBP10. LPS and PIP2 also caused congruent changes in the fluorescent spectrum of pyrene derived SEQID No:1 (QRLFQVKGRR), in which binding to these lipids, but not to PS, induces clustering of the peptide with resulting excimer fluorescence of the pyrene group. This was manifest as a large increase in fluorescence emission at 480 nm (data not shown).

After an initial decrease in fluorescence at low LPS to peptide ratios, the peptide fluorescence increases strongly, further suggesting insertion of the peptide-bound rhodamine-B into a more hydrophobic environment. Lipid A (the LPS derivative lacking polysaccharide) causes weaker, but detectable inhibition; whereas anionic lipids, like PS, have no inhibitory effect (data not shown). Thus, LPS is a more potent inhibitor than either LPA or PIP2, yet certain structural similarities between PIP2, LPA and LPS suggest that LPS binds gelsolin at the same site as PIP2 and LPA. When tested, the same types of assays that have documented binding of PPIs and LPA to gelsolin, all show that gelsolin binds LPS at least as strongly, and in some contexts with higher affinity than it binds PIP2, and the binding site is, in fact, localized to apparently to the same region in gelsolin where PIP2 and LPA bind.

FIG. 1D confirms the rhodamine studies, using a pyrene-derivatized gelsolin peptide, although larger amounts are needed, as compared to inhibition of pure gelsolin in aqueous solution. Binding to LPS induced clustering of the peptide with a resulting excimer fluorescence of the pyrene group, which was evident as a large increase in fluorescence emission at 480 nm. LPS, LPA, and PIP2 had similar abilities to induce excimer formation, whereas PS had no effect (data not shown).

The molecular structure of LPS and its complexes with proteins, such as gelsolin can be further defined by plotting neutron scattering intensity. For example, the neutron scattering intensity of LPS + SEQID No:1 (gelsolin peptide residues 160-169) is shown in FIG. 2. The linearity of the plot suggests that LPS forms a cylindrical structure, but that the addition of gelsolin alters the diameter of LPS, confirming that neutron scattering data permits structural analysis of the binding capability of LPS.

The consequence of LPS binding on gelsolin's actin severing capability is shown in FIG. 3. When added to purified human gelsolin, LPS inhibited 50% of the severing activity of the gelsolin and is a more potent inhibitor of either LPA (lysophosphatidic acid, which is a potent extracellular agonist) or PIP2. Lipid A, the LPS derivative lacking polysaccharide has a weaker but significant inhibitory effect, and nonspecific anionic lipids like PS have no effect. When added to whole human serum, LPS and PIP2 are also able to inhibit gelsolin activity, although larger amounts are needed as compared to inhibition of pure gelsolin in aqueous solution. LPA appears to be much less effective in serum than in pure solution, possibly because the greater lability of the lysolipid allows its more rapid partitioning into lipoprotein particles or Ca²⁺-mediated aggregates that are incapable of binding gelsolin. Gelsolin competes with LBP (LPS-binding protein) for LPS. Using a solid phase binding assay, nanomolar concentrations of gelsolin were shown to inhibit binding of ¹²⁵I-LBP to LPS coated wells by 69 ±6% (FIG. 3A). However, inhibition of LBP-LPS binding is not due to direct binding of LBP to gelsolin since ¹²⁵I-LBP does not bind to gelsolin-coated surfaces (FIG. 3B). Since plasma contains higher levels of gelsolin (150-300 µg/ml), than that which is found in LBP (3-10 µg/ml) {Lind *et al.,* 1988, *supra;* Mathison, 1992, *supra*}*,* these data, showing comparable LPS affinity by gelsolin and by LBP, indicate that gelsolin may play a role in LPS-buffering or presentation.

One result of gelsolin-LPS binding is inhibition of the actin binding activity of gelsolin, as well as the actin depolymerizing activity of blood serum. Inhibition by LPS of actin depolymerizing activity in serum is was shown to be due to its effect on gelsolin, because the actin-sequestering activity of Gc-globulin (or vitamin D-binding protein), which is the other component of the plasma actin scavenger system, was unaffected by LPS at concentrations at least up to 100 µM (data not shown). A 10-fold molar excess of LPS from *E. coli* (serotype 026:B6), *Salmonella enteritidis* (ATCC13076 strain) or *Pseudomonas aeruginosa* (serotype 10)(data not shown) effectively inhibited -50% of gelsolin's actin filament severing capability. When added to purified human gelsolin, LPS inhibits the severing activity of gelsolin at a rate that increases with increasing concentration of LPS present (FIG. 4A). When added to whole human serum, LPS and PIP2 were also able to inhibit gelsolin activity, although larger amounts were needed compared to inhibition of pure gelsolin in aqueous solution (FIG. 4B).

Measurement of the effects on blood serum severing function showed that the inhibitory efficiency varies with the bacterial species from which the LPS was released. For example, when several different LPS species were tested, the inhibitor effect was found to be *Klebsiella pneumoniae* (strain ATCC 15380, Sigma) *<Salmonella enteriditis* (Strain ATCC 13076, Sigma) *<Escherichia coli* (data not shown). On a molar basis, LPS has proven to be a more potent inhibitor of gelsolin actin severing capability than either LPA or PIP2. Lipid A had a weaker, but still significant inhibitory effect, and nonspecific anionic lipids like PS had no effect (data not shown).

Therefore, it can be further concluded, by both chemical and physical evidence, that gelsolin binds LPS through its lipid-binding domain, and that the binding of polyphosphoinositides and LPA to gelsolin strongly inhibits gelsolin's actin filament severing function. Neutron scattering measurements suggest that LPS added to buffers *in vitro* forms micelles, and its interaction with gelsolin and other proteins may be different when it is in other complex forms. LPA appeared to lack its effect in serum, as compared to pure solution, possibly because the greater lability of the lysolipid allows its more rapid partitioning into lipoprotein particles or Ca²⁺-mediated aggregates that are incapable of binding gelsolin.

Gelsolin may prevent LPS-induced cellular effects *in vitro.* LPS effects on cellular functions, including cytoskeletal actin remodelling and collagen-induced platelet activation by pathways independent of toll-like receptors (TLR), are neutralized by gelsolin and by a peptide based on gelsolin residues 160-169 (SEQID No:1) which comprises part of gelsolin's phosphoinositide binding site. When added to either cultured human aortic endothelial cells (HAEC) or primary rat astrocytes, LPS at relatively high concentrations rapidly induced disassembly of the actin cytoskeleton (see disrupted and diffuse phalloidin staining in FIGs. 5B and 5E). By comparison, however, abundant actin bundles are seen in the controls (FIGs. 5A and 5D). However, when the LPS was first incubated with an excess of plasma gelsolin, the thus bound LPS had no discernable effect on the actin cytoskeletons of either the endothelial cells or the astrocytes.

TLR-dependent NF-kB translocation in fibroblasts appears not to be blocked by gelsolin (see Example 3), whereas LPS effects on cellular functions, including cytoskeletal actin remodeling by pathways independent of toll-like receptors (TLR) are neutralized by gelsolin and by a peptide based on gelsolin SEQID No:1. In untreated control astrocytes, NF-κB was located in the cytoplasm (Figure 5G + K) and activation by LPS resulted in its translocation to the nucleoplasm (FIGs. 5I and 5M) similar to that induced by TNF-α (FIGs. 5H and 5L). However, the LPS-induced translocation of NF-κB (FIGs. 5I and 5M) was blocked by pre-incubation with gelsolin (FIGs. 5J and 4N). These results show a strong effect of LPS on plasma gelsolin function, and more importantly indicate that at least some effects of endotoxin *in vivo* are mediated or inhibited by plasma gelsolin.

Generalized coagulation dysfunction represents a common complication of LPS-induced septic shock syndrome. The effect of LPS on platelet function has reportedly depended on LPS concentration used to affect platelet function. It has be found that LPS at low concentration caused priming of platelets activation (Salat et al., Leuk. Lymphoma 33(1-2):25-32 (1999)), but LPS at 100-300 µg/ml inhibits platelets aggregation stimulated by collagen (Sheu et al., Br. J. Haematol. 103(1):29-38 (1998)). However, this LPS effect on collagen-induced platelet activation by pathways independent of toll-like receptors is neutralized by gelsolin. For example, when LPS was added alone, LPS strongly inhibited the aggregation and secretion of platelets induced by collagen (compare triangles to square symbols in FIG. 6). By comparison, the addition of gelsolin SEQID No:1 to LPS before it was added to the platelets, restored, in a peptide concentration-dependent manner, the normal collagen-induced activation and aggregation of the platelets. However, the gelsolin peptide (SEQID No:1) alone, as opposed to with LPS, had no effect on collagen-mediated platelet functions.

Therefore, FIG. 6 shows the protective effect of the gelsolin peptide SEQID No:1 (QRLFQVKGRR) (GLS 160-169) on LPS-mediated alteration of platelet function. These results show a strong effect of LPS on plasma gelsolin function and indicate that some of the pathogenic effects of endotoxin *in vivo* may be mediated, inhibited or even prevented by plasma gelsolin, or an active fragment thereof.

Since humans with sepsis have decreased plasma gelsolin concentrations (Suhler *et al.,* 1997, *supra),* the effect of LPS administration on gelsolin levels can be studied in recognized mouse models. Intraperitoneal administration of *P. aeruginosa* LPS (serotype 10) caused a dose-dependent reduction in plasma gelsolin levels after 24 hours. The changes in gelsolin concentrations in response to LPS appear to be specific, because plasma albumin . levels did not change. The modest reduction in plasma gelsolin level was consistent with the relatively low mortality (35% by 7 days after treatment) of mice receiving 40 mg/kg of *P. aeruginosa* LPS. In contrast, mice challenged with LPS from *E. coli* (serotype 055:B5) at 25 mg/kg decreased their plasma gelsolin values by 50% within 24 hours after challenge, and had a 100% mortality rate by 7 days (data not shown).

The mice were administered human recombinant plasma gelsolin (GSN) subcutaneously, immediately after challenge with *E. coli* LPS, and daily thereafter for three additional doses. The mortality rate was then compared with those treated with saline solution alone, or with bovine serum albumin (BSA). The gelsolin amounts administered by this route restored plasma levels in LPS-treated animals to 200 µg/ml, which is slightly greater than the baseline concentrations (data not shown). However, mice treated with plasma gelsolin have markedly improved survival compared to saline or BSA controls (FIG. 7). At Day 7, the surviving mice (all in the GSN group) all appeared to have completely recovered and exhibited no signs of distress.

The tight and selective binding of gelsolin to LPS has several implications for the function of plasma gelsolin and may suggest methods to counteract the toxic effects of LPS. The wide distribution of a extracellular gelsolin protein in eukaryotic species, including humans, and its binding to a variety of lipopolysaccharide endotoxins suggests that it is a component of the innate immune system (Beutler et al., Crit. Care Med. 29 (supplement) S2-S7 (2001)). The findings presented in this disclosure support the concept of a strong clinical correlation between lowered gelsolin levels due to primary injury and susceptibility to secondary complications may result from a loss of buffering of inflammatory lipid mediators. The lack of reported human antibodies directed against gelsolin indicates that this normal plasma constituent has a high degree of structural conservation between individuals. Therefore, it is a preferred embodiment of the present invention to provide gelsolin or a functionally equivalent fragment thereof, for use as a medicament in a method of preventing, neutralizing or reducing endotoxemia or endotoxin-induced septic shock in a patient *in vivo,* wherein the patient is subject to or susceptible to gram negative bacterial infection.

Transgenic constructs may be generated comprising an isolated nucleic acid comprising the nucleic or amino acid sequence of gelsolin or an active fragment thereof. Suitable vectors include, but are not limited to, plasmids containing a sense or antisense strand placed under the control of the strong constitutive promoter or under the control of an inducible promoter. Methods for the generation of such constructs are well known in the art once the sequence of the desired gene is known. Suitable vector and gene combinations will be readily apparent to those of skill in the art.

A "vector" is a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid or a virus. The term should also be construed to include non-plasmid and non-viral compounds, which facilitate transfer of nucleic acid into cells, such as, for example, polylysine compounds, liposomes, and the like. "Expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an in vitro expression system. Expression vectors include all those known in the art, such as cosmids, plasmids (*e.g.,* naked or contained in liposomes) and viruses that incorporate the recombinant polynucleotide.

The nucleic acid encoding the gelsolin peptide can be duplicated using a host-vector system and traditional cloning techniques with appropriate replication vectors. A "host vector system" refers to host cells, which have been transfected with appropriate vectors using recombinant DNA techniques. The vectors and methods disclosed herein are suitable for use in host cells over a wide range of eukaryotic organisms. This disclosure also encompasses cells transformed with the replication and expression vectors, using methods known in the art. Indeed, a gene encoding the modulating nucleic acid, such as the nucleic acid sequence encoding gelsolin can be duplicated in many replication vectors, and isolated using methods described, *e.g.,* in Sambrook *et al.,* 1989.

The selected gene, made and isolated using the above methods, can be directly inserted into an expression vector, such pcDNA3 (Invitrogen) and inserted into a suitable animal or mammalian cell. The gene or gene fragment, such as the purified nucleic acid molecule encoding, *e.g*., gelsolin, may be introduced into the cell and expressed. A variety of different gene transfer approaches are available to deliver the gene or gene fragment encoding the modulating nucleic acid into a target cell, cells or tissues.

As used herein, "recombinant" is intended to mean that a particular DNA sequence is the product of various combination of cloning, restriction, and ligation steps resulting in a construct having a synthetic sequence that is indistinguishable from homologous sequences found in natural systems. Recombinant sequences can be assembled from cloned fragments and short oligonucleotides linkers, or from a series of oligonucleotides. As noted above, one means to introduce the nucleic acid into the cell of interest is by the use of a recombinant expression vector. "Recombinant expression vector" is intended to include vectors, capable of expressing DNA sequences contained therein, where such sequences are operatively linked to other sequences capable of effecting their expression. It is implied, although not always explicitly stated, that these expression vectors must be replicable in the host organisms, either as episomes or as an integral part of the chromosomal DNA. Suitable expression vectors include viral vectors, *e.g*., adenoviruses, adeno-associated viruses, retroviruses, cosmids and others, typically in an attenuated or non-replicative form. Adenoviral vectors are a particularly effective means for introducing genes into tissues.in *vivo* because of their high level of expression and efficient transformation of cells, both *in vitro* and *in vivo.*

Accordingly, when reference is made herein to "administering" gelsolin or a functionally equivalent peptide fragment thereof to a patient, it is intended that such methods include not only delivery of an exogenous composition to the patient, but also methods for increasing expression levels of the gelsolin within the patient. Expression levels of the gene or nucleotide sequence inside a target cell are capable of providing gene expression for a duration and in an amount such that the nucleotide product therein is capable of providing a therapeutically effective amount of gene product or in such an amount as to provide a functional biological effect on the target cell. By "gene delivery" is meant transportation of a composition or formulation into contact with a target cell so that the composition or formulation is capable of being taken up by means of a cytotic process into the interior or cytoplasmic side of the outermost cell membrane of the target cell, where it will subsequently be transported into the nucleus of the cell in such functional condition that it is capable of achieving gene expression.

By "gene expression" is meant the process, after delivery into a target cell, by which a nucleotide sequence undergoes successful transcription and translation such that detectable levels of the delivered nucleotide sequence are expressed in an amount and over a time period that a functional biological effect is achieved. "Gene therapy" encompasses the terms gene delivery and gene expression. Moreover, treatment by any gene therapy approach may be combined with other, more traditional therapies.

By "patient" or "subject" is meant any vertebrate or animal, preferably a mammal, most preferably a human, that is affected by or susceptible to endotoxin-induced sepsis or inflammation-induced pulmonary microvascular dysfunction. Thus, included within the present invention are animal, bird, reptile or veterinary patients or subjects, the intended meaning of which is self-evident. The present disclosure may be useful in such a patient for the treatment or prevention of the following, without limitation: septic shock, pathogenesis relating toa severe burn, myocardial infarction, acute hepatic failure, myonecrosis, acute lung injury, and adult respiratory distress - multiple organ dysfunction syndrome (ARDS/MODS).

The gelsolin or gelsolin active fragment (SEQID No:1) may be designed by mimetics for synthetic production. The designing of mimetics to a pharmaceutically active compound is a known approach to the development of pharmaceuticals based on a "lead" compound. This might be desirable where the active compound is difficult or expensive to synthesize or where it is unsuitable for a particular method of administration. Mimetic design, synthesis and testing is generally used to avoid randomly screening large number of molecules for a target property, see, *e.g.,* US Patent Nos. 5,071,773; 5,750,353; 5,925,529; 5,744,303; 5,569,588; and 5,407,821.

The disclosure further provides an assay for determining agents, which bind to, neutralize or inhibit lipopolysaccharides, LPS-binding proteins, or other factors in a sequence of events leading to the onset of endotoxemic sepsis or inflammation-induced pulmonary microvascular dysfunction, thereby reducing, modulating or preventing such pathologies. Such an assay comprises administering an agent under test to the cells or model animals, such as those described herein at low cell density, and monitoring the onset or severity of sepsis or microvascular dysfunction. A further assay according to the disclosure comprises administering the agent under test to photoreceptor cells at high cell density, and monitoring the onset or severity of sepsis or microvascular dysfunction. Agents may thus be selected which effectively reduce, inhibit, neutralize or prevent sepsis, microvascular dysfunction, ARDS or the like. The agents thus selected, are also intended to be a part of the present disclosure.

In yet another aspect of the disclosure, sensitivity of gelsolin levels *in vivo* are used as a biomarker for measuring disease severity in sepsis or inflammation, or for determining levels of bacterial endotoxins.

In accordance with the present disclosure, the gelsolin, or fragment thereof, intended to bind to, neutralize or inhibit lipopolysaccharides, LPS-binding proteins, actin-binding proteins or other factors in a sequence of events leading the onset of endotoxemic sepsis, thereby reducing, modulating or preventing such sepsis or inflammatory response, as described above, when used in therapy, for example in the treatment of an endotoxemic patient or one infected with a gram negative bacteria, can be administered to such a patient either alone or as part of a pharmaceutically acceptable composition, and optionally with a preservative, diluent, and the like. They may further be administered in the form of a composition in combination with a pharmaceutically acceptable carrier or excipient, and which may further comprise pharmaceutically acceptable salts. Examples of such carriers include both liquid and solid carriers, such as water or saline, various buffer solutions, cyclodextrins and other protective carriers or complexes, glycerol and prodrug formulations. Combinations may include other pharmaceutical agents.

The term "pharmaceutically acceptable salts" refers to physiologically and pharmaceutically acceptable salts of the compounds of the invention: *i.e.,* salts that retain the desired biological activity of the parent compound and do not impart undesired toxicological effects thereto.

Various methods of "administration" of the therapeutic or preventative agent can be used, following known formulations and procedures. Although parental administration is described herein and is generally preferred, systemic or targeted administration may also be employed under suitable circumstances. Compounds or compositions suitable for parenteral injection may comprise physiologically acceptable sterile aqueous or nonaqueous solutions, dispersions, suspensions, or emulsions, and sterile powders for reconstitution into sterile injectable solutions or dispersions. Examples of suitable aqueous and nonaqueous carriers, diluents, solvents, or vehicles include water, saline, buffered saline, dextrose, ethanol, glycerol, polyols, and the like, and suitable mixtures thereof. Proper fluidity can be maintained, for example, by the use of a coating, such as lecithin, by the maintenance of the required particle size in the case of dispersions and by the use of surfactants. These compositions may also contain adjuvants, such as preserving, wetting, emulsifying, and dispensing agents. Sterility can be ensured by the addition of various antibacterial and antifungal agents. It may also be desirable to include isotonic agents, for example sugars, sodium chloride and the like. Prolonged absorption of the injectable pharmaceutical form can be brought about by the use of agents delaying absorption, for example, aluminum monostearate and gelatin.

Persons of ordinary skill can easily determine optimum dosages, dosing methodologies and repetition rates. Repetition rates for dosing can be readily estimated based upon measured residence times and concentrations of the drug in bodily fluids or tissues. Amounts and regimens for the administration of gelsolin or related actin-binding compounds can be determined readily by those with ordinary skill in the clinical art of treating actin-related disorders, tissue injury and inflammation. Generally, the dosage of gelsolin or related actin-binding compound treatment will vary depending upon considerations, such as: age; health; conditions being treated; kind of concurrent treatment, if any, frequency of treatment and the nature of the effect desired; extent of tissue damage; gender; duration of the symptoms; and, counter-indications, if any, and other variables to be adjusted by the individual physician. Dosage can be administered in one or more applications to obtain the desired results (see, *e.g.,* dosages proposed for human therapy by Rothenbach *et al.,* 2004, *supra).*

The dosage can be calculated in the following manner. If the normal blood gelsolin concentration is 2.4 µM (2.4 µmol/l), and the normal blood DBP concentration is 5 µM (5 µmol/l). Thus, the total blood actin-binding capacity (ABC) is approximately 7.5 µmol/l. The blood volume is 6% of the body weight, hence a 70 kg person has about 4.2 liters of blood and as a result, 4.2 1 x 7.5 µmol/1 = 31.5 µmols ABC. Since gelsolin is distributed throughout the extracellular space (which is -10% of body weight), the body contains 7.5 x 7 = 52.5 µmols gelsolin ABC.

It may be desired to administer between 32 and 53 µmols of gelsolin (or 0.46 µmol/kg body weight) to cover total complexing or depletion of endogenous gelsolin or other actin binding compounds. Plasma concentration of endogenous gelsolin in normal, uninjured humans is 211 µg/ml, and in rabbits, 368 µg/ml (Lind *et al.,* 1986, *supra).* Since 0.425 mg of actin is equal to 1 µmol, and since there is 4.86 mg actin per gram of skeletal muscle, each gram of muscle contains about 11.3 µmol actin. Thus, 4.6 g of muscle destruction could neutralize total body gelsolin or other actin binding compounds. However, because the toxic effects of actin are presumably local (e.g., inhibition of clot lysis), sequestered or kinetically determined (e.g., actin permeates an organ faster than binding proteins neutralize it), it is likely that a theoretically minimum dose will have to be adjusted upward in order to achieve kinetically favorable therapeutic effects. The kinetic effect can be important since there is apparently slow equilibration between extravascular and blood compartments acutely (Smith *et al.,* 1988, *supra).* Conversely, a therapeutically effective state, capable of breaking up local deposits of actin, may be achievable only by a transient pulse of a high concentration of gelsolin or actin-binding molecules.

Instead of administering the gelsolin or gelsolin peptides or other binding compounds directly, they can also be produced in the target cells by expression from an encoding gene introduced into the cells, *e.g.,* in a viral vector. The vector could be targeted to the specific cells to be treated, or it could contain regulatory elements, which are switched on more or less selectively by the target cells.

By "therapeutically effective" as used herein, is meant that amount of composition that is of sufficient quality and quantity to neutralize, ameliorate, modulate, or reduce the. cause of or effect of endotoxin caused sepsis or inflammation. Because the gelsolin lipid-binding activity is Ca²⁺-mediated, it is assumed when the term "therapeutically effective," with regard to administration of gelsolin or the functionally equivalent peptide fragment thereof, that Ca²⁺ or other necessary divalent ion is present at the levels necessary to actrivate the gelsolin molecule. By "ameliorate," "modulate," or "reduce" is meant a lessening or reduction or prophylactic prevention of the detrimental effect of the disorder in the patient receiving the therapy, thereby resulting in "protecting" the patient. A "sufficient amount" or "effective amount" or "therapeutically effective amount" of an administered composition is that volume or concentration which causes or produces a measurable change from the pre-administration state in the cell or patient. The subject is preferably a human patient, however, it can be envisioned that any animal with endotoxemia or sepsis can be treated using the present invention. As used herein, the terms "treating" and "treatment" are intended to include the terms "preventing" and "prevention." One embodiment of the present invention includes the use of gelsolin, or functionally equivalent peptide fragment thereof, in the manufacture of a medicament for preventing, neutralizing or reducing endotoxemia or endotoxin-induced septic shock in a patient.

The following specific, but nonlimiting, examples describe in detail the preparation of exemplary injectable compositions and methods using them for tissue engineering. Reference is made to standard textbooks of molecular biology that contain definitions and methods and means for carrying out basic techniques, encompassed by the present invention. See, for example, Maniatis et al., Molecular Cloning: A Laboratory Manual, Cold Spring Harbor Laboratory, New York (1982) and Sambrook et al., Molecular Cloning: A Laboratory Manual, Cold Spring Harbor Laboratory, New York (1989) and the various references cited therein. It will be apparent to those skilled in the art that many modifications, both to materials and methods, may be practiced.

### EXAMPLES

### Example 1 - Gelsolin Binding and Severing Capabilities

*Binding activity* - To provide chemical and functional evidence that gelsolin binds LPS through its lipid-binding domain, a solid phase assay was conducted in which radiolabeled LPS was added to wells coated with different amounts of rhodamine-B-labeled SEQID No:1 (rhodamine-B-QRLFQVKGRR, derived from the PIP2-binding site of gelsolin and termed "PBP10"), or to a truncated peptide (rhodamine-B-QRL), in the absence or presence of unlabeled LPS, respectively.

SEQID No:1 (QRLFQVKGRR) gelsolin residues 160-169, and QRL peptides, were prepared by solid phase peptide synthesis on p-benzyloxybenzyl alcohol/polystyrene resin using alpha-Fmoc protection chemistry and carbodiimide/N-hydroxybenzotriazole coupling as taught by Cunningham et al., J Biol Chem 276:43390-9 (2001)). The side chains were protected as follows: Arg (Pmc), Gln (Trt), Lys (Boc). To couple fluorophores onto these peptides, ester derivatives of each fluorophore were created and linked directly to the N-terminus of the peptides on the solid phase support. After coupling, the peptides were cleaved from the solid support with trifluoroacetic acid and phenol (95:5, v/w). All peptides were purified by reverse phase HPLC on a silica C-18 column using a 20-60% acetonitrile gradient in 0.1 % trifluoroacetic acid and dried (Cunningham *et al., 2001, supra*)*.*

To determine the interaction of LPS with gelsolin and fluorescence peptides derived from gelsolin PIP2 binding site, the fluorescence of rhodamine B-peptide SEQID No:1 or emissions spectra of pyrene-peptide SEQQID No:1 (QRLFQVKGRR) (λem 590 nm, λex 560 nm, or λem 365-550 nm, λex 343 nm, respectively) was measured fifteen minutes after addition of various concentrations of LPS *(Escherichia coli* serotype O26:B6, Sigma), PIP2, LPA or PS added to 2 µM solutions of the peptides in buffer A (10 mM TRIS, 10 mM MES (2-morpholinoethane-sulfonic acid), pH 7.0. Since the surface concentration of the peptides bound to the lipids will become much higher than their bulk concentration, peptide binding was demonstrated by changes of either rhodamine-B fluorescence or formation of pyrene-excimers, with a shift of fluorescence emission from the monomer wavelength at 378 nm to the excimer emission at 473 nm. Therefore, to determine changes of gelsolin OD at 280 nm, different amounts of LPS, LPA and PS were added to recombinant human gelsolin (rhGLS) solutions and OD emission spectra were recorded using spectrophotometer Genesys 6 (Thermo-Spectronic, USA).

*Solid Phase Assay* - Tritium-labeling of LPS was done by a modification of the procedure of Watson and Riblet (Watson et al., J. Immunol 114:1462-8 (1975)). A sample (2 mg) of LPS from *Salmonella minnesota* Re595 (Sigma Chemical Co., St. Louis, MO) was oxidized (150 minutes, 20°C) with sodium periodate (3x10⁻² M). After destruction of the oxidant with 1.0 M ethylene glycol, aldehyde groups were reduced (18 hours at 4°C) with an ice-cold solution of NaB [³H]₄ (0.46 GBq, 481 GBq/mmole, wherein GBq refers to a unit of radioactivity) (Amersham-Pharmacia Biotech, Buckinghamshire, England) in 200 µl of ice-cold borate buffer (0.05 M, pH 9.5). Excess sodium borohydride was destroyed with 5 µl of acetic acid. After two washings (centrifugation at 100,000 x g for 15 minutes) in 400 µl of an ice-cold water-ethanol mixture (1:1 by vol.), the radiolabeled [³H]-LPS (9x10⁵ cpm/µg; 2x103 cpm/pmole) was stored at -20°C until used.

To measure [³H] labeled-LPS-binding, polystyrene ELISA-type microplates (96 wells) were coated with PBP10 or rhodamine-B-QRL (RhB-QRL) by incubation for 18 hours at 4°C with a solution (100 µl, 500 pmole/well) in saline. After three washings with 100 µl saline, the wells were then incubated at room temperature with [³H]-LPS (360,000 c.p.m.), in the presence or absence of unlabeled LPS (20 µg/well), in a binding medium (100 µl) containing bovine serum albumin (50 µg) in saline. After 3 hours of incubation, the plates were washed three times with 100 µl saline washes, and the remaining bound radioactivity was measured.

The results are shown in FIG. 1A of the solid phase assay, wherein radiolabeled LPS was added to wells coated with different amounts of PBP10 peptides ± cold LPS. The results in the absence of unlabeled LPS is shown as open circles, while the presence of unlabeled LPS is shown as open triangles: As shown, LPS saturably bound PBP10, and resulted in substantial inhibition of labeled LPS binding in the presence of excess LPS. By comparison, the truncated peptide (rhodamine-B-QRL) did not bind LPS.

FIG. 1B, showing the optical density (OD) of gelsolin in solutions containing varying amounts of LPS or LPA, demonstrates the interaction of LPS or PIP2 with intact gelsolin. Both LPS and PIP2 induced identical concentration-dependent fluorescence quenching of PBP10, indicating insertion of the peptide-bound rhodamine into a more hydrophobic environment. LPA also diminished the absorbance of gelsolin, but PS had no effect. LPS and PIP2 also caused congruent changes in the fluorescent spectrum of pyrene-QRLFQVKGRR (Bucki et al., Biochemistry 40:15752-61 (2001)) in which binding to these lipids - but not to PS - induced clustering of the peptide with resulting excimer fluorescence of the pyrene group, manifest as a large increase in fluorescence emission at 480 nm (data not shown).

The fluorescence of rhodamine-B-SEQID No:1 (QRLFQVKGRR) was also changed by LPS as shown in FIG. 1C. There was also no fluorescence change after adding LPS to a control peptide with the sequence rhodamine B-QRL (RhB-QRL, data not shown).

Interaction of LPS and gelsolin was examined using rhodamine B- and pyrene-labeled SEQID No:1 (QRLFQVKGRR). The effect of LPS on gelsolin was determined in terms of the rhodamine-B fluorescent changes of PBP10 peptide in the presence of different lipids (FIG. 1C); and the fluorescence emission spectra of pyrene-labeled SEQID No:1 (2 µM) in the presence of LPS from *Escherichia coli* (1.2 µM), PIP2 (0.82 µM) and LPA (0.82 µM) (FIG. 1D) The results using the pyrene-derivatized gelsolin peptide confirmed the LPS induced clustering of the peptide with resulting excimer fluorescence of the pyrene group, which was evident as a large increase in fluorescence emission at 480 nm. LPS, LPA, and PIP2 had similar abilities to induce excimer formation, whereas PS had no effect (data not shown).

Alternative assays could also be used to confirm the binding characteristics of LPS to gelsolin. One test uses radiolabeled LPS and assays for gelsolin by affinity chromatography using immobilized anti-gelsolin antibody (a similar assay was used to detect binding of ³²PIP2 to gelsolin (Janmey et al., Nature 325:362-364 (1987). Glass tubes containing phosphatidylcholine (PC) (0.3 mg) alone or mixed with the material to be tested (gelsolin, gelsolin half-mutant lacking PIP2 binding site, PBP10 peptide, etc) is evaporated to dryness. After sonication with a solution of BSA (60 µl, 1 mg/ml in 0.15 M NaCl), the vesicles are incubated (2 hours, 20° C) under gentle rotation, with [³H]-LPS. Then the density is adjusted to 1.185 g/ml by addition of 350 µl of a solution of 1.1% NaCl and 46% NaBr. A discontinuous gradient will be prepared by sequential addition of 23% NaCl (700 µl) and the radioactive suspension (700 µl), 20.5% NaCl (700 µl), 16.5% NaCl (700 µl), and 8% NaCl (200 µl). After centrifugation (65,000 g, 90 minutes), the radioactivity of collected fractions is measured by liquid scintillation. Vesicles with bound radio-labeled LPS are recovered at the top (1 ml) of the density gradient, whereas unbound [³H]-LPS was found at the bottom. The LPS-binding index of a compound is defined as the radioactivity recovered at the top (1 ml) of the density gradient, expressed as a percentage of the total radioactivity recovered.

*Measurement of complexes bv neutron scattering* - The molecular structure of LPS and its complexes with proteins can be measured at nm resolution by X-ray and neutron scattering, as well as by electron and atomic force microscopy. FIG. 2 shows preliminary neutron scattering measurements of the effects of gelsolin peptide SEQID No: I on LPS structure.

The linearity of the plot of ln(QI(Q)) vs. Q2 suggests that LPS forms a cylindrical structure under the conditions used, consistent with an earlier neutron scattering study (Hayter et al., J. Biol. Chem. 262:5100-5105 (1987)). The small, but significant increase in calculated diameter of LPS when the gelsolin peptide is added, suggests an alteration of the diameter. At present these data only serve to confirm that sufficiently good scattering data can be gathered for structural analysis. At present, the calculated diameter (36 nm) is greater than that which has been previously reported for pure LPS micelles, and the difference in size may reflect another structural feature. In additional experiments with more variations in gelsolin/LPS or peptide, LPS ratios will reveal if there are significant changes in LPS particle structure when these peptides bind LPS.

Further neutron scattering studies can be done at Argonne National Laboratories where the present data were taken. Each such measurement takes about 4 to 6 hours of beam time, so specimens are limited to selected conditions in which DLS or electron microscopy suggests novel structures formed by LPS and gelsolin, and where the biological activity of LPS is inhibited. Transmission electron microscopy of these complexes will also be done. If lamellar ribbon-like structures, similar to those formed by PEP2 and gelsolin, geometrically the head groups may be occluded in the interior of the ribbon, and if similar structures are seen when adding gelsolin to LPS, the removal of the charged groups of LPS from the surface of the structure may relate to a loss of its cellular effects.

### Example 2 - Gelsolin competes with LPS-binding protein (LBP) for LPS

To determinate whether gelsolin can effect an action similar to LBP-mediated incorporation of LPS into blood lipoprotein an experiment is utilized, in which repartition of Bodipy- or ³H- labeled-LPS to reconstituted HDL (R-HDL) or plasma lipoproteins is measured. R-HDL is prepared by mixing apo A-1, egg PC, cholesterol and cholate at molar ratio 1:80:4:80 followed cholate dialysis as previously described by Yu *et al.,* 1997, *supra.* The fluorescence of Bodipy-LPS in micelles is quenched, but quenching is relieved and fluorescence increases upon movement of Bodipy-LPS monomers out of aggregates. An increase in fluorescence is observed when both gelsolin and R-HDL are mixed with Bodipy-LPS provides evidence for gelsolin-depended transfer of LPS to R-HDL, similar to that seen with LBP. Pure LPS and various amounts of gelsolin are added to observe if gelsolin alone changes the Bodipy-LPS fluorescence, since any such change will have to be subtracted from the initial fluorescence after adding lipoproteins. The rate and extent of fluorescence change after lipoprotein addition offers a means for monitoring exchange of LPS into the lipoprotein particles. For purified systems, lipoprotein size is also measured by dynamic light scattering. Since DLS data are heavily weighted by the largest particles, the average hydrodynamic diameter will be nearly equal to that of lipoproteins alone, and any change indicates that the particles are fragmented or fused by gelsolin/LPS.

Since fluorescence measurements are strongly perturbed in plasma or serum, partitioning is examined of ³H-labeled-LPS to blood plasma lipoprotein fractions in gelsolin-depleted samples using anti-gelsolin coated sepharose 2B beads as done previously by Janmey *et al.,* 1987, *supra,* or in plasma of gelsolin-null mice after adding back various concentrations of gelsolin. Because the interactions of two other known plasma proteins: a soluble form of CD14 receptor (sCD14) and plasma LBP (their plasma concentration is 2-6 µg/ml and 3-10 µg/ml respectively), may interfere with this assay, these proteins are also be removed by sepharose 2B beads coated with their respective antibodies.

To determine competition between gelsolin and LPS-binding proteins (LPB), a high affinity carrier for LPS, the LPS-coated wells (1 nmole/per well of LPS Re-595 from *Salmonella minnesota)* were saturated by incubation (2 hours, 20° C) with gelsolin 2 mg/ml in RPMI), washed and preincubated with different amount of recombinant human gelsolin (0-2 ng/well) in gelsolin (0.5 mg/ml) containing RPMI. Then, 10 µl of ¹²⁵I-LPB (6 x 10⁴ cpm) were added, and the plates were incubated for 90 minutes at 37°C, then washed six times with RPMI. After that, the bound radioactivity was measured. Similarly, gelsolin-coated wells were used to demonstrate whether rhGLS directly interacts with ¹²⁵I-LPB.

It was shown that nanomolar concentrations of gelsolin inhibited the binding of ¹²⁵I-LBP (LPS-binding protein) to LPS coated wells by 69 ±6% (FIG. 3A). Moreover the inhibition of LBP-LPS binding was not due to the direct binding of LBP to gelsolin, since ¹²⁵I-LBP did not bind to gelsolin-coated surfaces (FIG. 3B). Because plasma contains higher levels of gelsolin (150-300 µg/ml), than the gelsolin in LBP (3-10 µg/ml) these data, showing comparable affinity of gelsolin and LBP for LPS, demonstrate that gelsolin plays a role in LPS-buffering or presentation.

### Example 3 - Gelsolin's actin filament severing activity and effect of LPS

To determine gelsolin's severing activity, rates of fluorescence decrease were evaluated during depolymerization. Monomeric G-actin was prepared from an acetone powder of rabbit skeletal muscle according to previously published methods (Spudich et al., J. Biol. Chem 216:4866-4871 (1975)). The non-polymerizing solution contained 2 mM TRIS, 0.2 mM CaCl₂, 0.5 mM ATP (adenosine triphosphate), 0.2 mM DTT (dithiothreitol) pH 7.4. Actin was polymerized by adding 150 mM KCl and 2mM MgCl₂ to the G-actin solutions and incubating for 1 hour at room temperature.

For F-actin severing assays, in general, a small volume (typically 5 µl) of the gelsolin composition is added to a similar volume of pyrene-labeled F-actin at a concentration adjusted to provide an actin:gelsolin ratio of approximately 10:1 (typically 1 µM gelsolin and 10 µM actin). This mixture is the diluted to 0.5 µM in a larger volume of F-actin buffer, and the filament severing efficiency is calculated from the initial slope of the fluorescence change. This reflects the rate of depolymerization that, in turn, is proportional to the number of cuts made by gelsolin. The LPS/gelsolin reaction can be done under any ionic condition, and the addition to F-actin requires the presence of ≥µM Ca²⁺ to activate the gelsolin. However, since the severing reaction requires only a few seconds, and the effects of Ca²⁺ on lipid aggregate size is slower and, at least for gelsolin/PIP2 does not reverse any complexes formed under other ionic conditions, it is possible, to a large extent, to alter the ionic conditions at which gelsolin binds LPS. The actin nucleation reaction is similar except that in the latter case, the gelsolin/LPS mixture is added to a more dilute solution of G-actin (typically, 1 or 2 µM) in low ionic strength buffer followed immediately by addition of 150 mM KCl and 2 mM MgCl₂ to initiate polymerization.

Any difference observed for binding of LPS to gelsolin or PBP10 by fluorescence assays in the present invention are also evaluated by the filament severing and nucleation tests.

As shown, recombinant human gelsolin (rhGLS) (FIG. 4A) and blood serum (FIG. 4B) severing activity was measured in 0.4 µM pyrene-labeled F-actin samples (polymerized with 50% pyrene-labeled G-actin), after adding gelsolin (16 nM)/serum (2.5 µl), or their combination with one of the following: LPS (from different species), PIP2 (phosphatidylinositol-4,5-bisphosphate), LPA (lysophosphatidic acid), lipid A (the LPS derivative lacking polysaccharide) or PS (phosphatidylserine). The fluorescence intensity of F-pyrene actin was then monitored for 10 minutes, and the severing activity was calculated based on decreasing fluorescence. Severing activity was determined from a decrease in rate of fluorescence during depolymerization, and the depolymerization rate was calculated from the slope of the early decrease in fluorescence.

When added to purified human gelsolin, 0.02 µM LPS (100 ng/ml) inhibited 50% of the severing activity of 0.016 µM gelsolin, and severing capability was 90% inhibited by a 10-fold increase to 0.1 µM LPS (FIG. 4A). On a molar basis, LPS was found to be a more potent inhibitor than either LPA or PIP2. A 10-fold molar excess of LPS from *E. coli* (serotype 026:B6) (FIG. 4A), *Salmonella enteritidis* (ATCC13076 strain) or *Pseudomonas aeruginosa* (serotype 10)(data not shown) effectively inhibited the ability of gelsolin to sever actin filaments. Lipid A had a weaker but still significant inhibitory effect, and nonspecific anionic lipids like PS had no effect (data not shown). When added to whole human serum (FIG. 4B), LPS and PIP2 were also able to inhibit gelsolin activity, although larger amounts were needed compared to inhibition of pure gelsolin in aqueous solution.

### Example 4 - Gelsolin prevents LPS-induced cellular responses to LPS in vitro

In light of the findings in the previous Examples, and using the materials prepared in above, the effect of the gelsolin binding by LPS was studied to determine the effect on cellular responses *in vitro.* Cell cultures of human aortic endothelial cells (HAEC) and rat primary astrocytes were used to evaluate the effect of LPS on actin. Both of these cell types have mCD14 (membrane bound C14) and TLR LPS receptors (Faure, et al., J. Biol. Chem. 275:11058-11063 (2000); Bsibsi, et al., J. Neuropathol. Exp. Neurol. 61:1013-1021 (2002); Bowman, et al., Glia 43:281-291 (2003)).

Rat primary astrocytes were obtained from prenatal rats and maintained for 14 days in culture before use. Embryos (E17-E19) were removed by caesarian section from a Sprague-Dawley rat and the hippocampi were surgically removed. The tissue was digested in trypsin/DNAse at 37°C, centrifuged (1000g x 5 min) and filtered to derive suspensions from each pup. Cells were grown in an incubator at 37°C and 5% CO₂ in DMEM (Dulbecco's Modified Eagle's Medium; BioWhittaker/Cambrex BioScience, East Rutherford, NJ), supplemented with Ham's F12 (Sigma-Aldrich, St. Louis, MO) and 5% fetal bovine serum (FBS) (Hyclone, Logan, Utah) for 7 days, followed by an additional 7 days in Neurobasal medium (Gibco, Grand Island, NY) also supplemented with 5% FBS, 2 mM 1-glutamine, 50 µg/ml streptomycin and 50 U/ml penicillin. In each experiment, the medium was changed to serum-free at 6-12 hours prior to addition of LPS, TNF-α, or gelsolin.

HAECs (primary human aortic endothelial cells) (ATCC, Rockville, MD or Clonetics, San Diego, CA), will grow to confluence through passages 5-9 in endothelial cell growth medium supplemented with 2% fetal bovine serum, 10 µg/liter human recombinant epidermal growth factor, 1 mg/liter hydrocortisone, 50 µg/ml gentamicin, 50 ng/ml amphotericin-B, in a 37° C humidified atmosphere of 95% air:5% CO₂.

HAECs and astrocytes cultures were incubated for 10 minutes in medium containing 10 µg/ml LPS alone, or LPS that had been pre-incubated with 0.16 mg/ml of human gelsolin. Cultures were fixed with ice-cold methanol and immunostained for F-actin with fluorescein isothiocyanate (FITC)-labeled phalloidin (Molecular Probes, Eugene, OR). In astrocyte cultures, NF-κB translocation was manipulated by a 2 hour incubation in serum-free medium containing one of the following: 10 ng/ml TNF-α, 10µg/ml LPS alone, or LPS that had been preincubated with 0.16 mg/ml human gelsolin. Location of transcription factor nuclear factor-kappaB (NF-κB) was observed using a monoclonal antibody to NF-κB (Molecular Probes), and cell nuclei were detected by counter-staining with 4',6-diamidino-2-phenylindole dihydrochloride (DAPI) (Sigma).

Gelsolin binding to LPS also influenced *in vitro* cellular responses to LPS, protecting platelts, aortic endothelial cells (HAEC), as well as primary rat astrocytes, against LPS. At relatively high concentrations, LPS rapidly induced the disassembly of the actin cytoskeleton as seen in the disrupted and diffuse phalloidin staining in FIGs. 5B and 5E, compared to the abundant actin bundles seen in the controls (FIGs. 5A and 5D). However, when first incubated with an excess of plasma gelsolin, LPS had no discernable effect on the actin cytoskeletons of either HAEC or astrocytes.

More specifically, FIGs. 5A-5M show that LPS-induced-disruption of cellular responses was inhibited by pre-incubating the LPS with plasma gelsolin. Briefly, immunostaining for F-actin with phalloidin (FIGs. 5A-5F) was used to demonstrate that plasma gelsolin prevented LPS-induced actin cytoskeleton disassembly. Cultured human aortic endothelial cells (FIGs. 5A-5C) and primary rat astrocytes (FIGs. 5D-5F) were treated with 2 µM LPS (FIGs 5B and 5E), or with 2 µM LPS and 1.8 µM gelsolin (FIGs. 5C and 5F). When the endothelial cells and astrocytes were exposed to LPS (10 µg/ml), disassembly of the actin cytoskeleton was readily induced as seen in the disrupted and diffuse phalloidin staining in FIGs. 5B and 5E, as compared to the abundant actin bundles in controls (FIGs. 5A and 5D). Control cells are shown in FIGs. 3A and 5D.

LPS is known to activate NF-κB, triggering induction of pro-inflammatory cytokine genes (see, Saura et al., J. Neurochem, 85:14S5-67 (June 2003)). However, in the astrocytes the addition of 10 ng/ml TNF-α (FIGs. 5H and 5L), or 2 µM LPS (FIGs. 5I and 5M), caused NF-kB translocation to the nucleoplasm of the nucleus, as compared with control astrocytes, in which the NF-κB locates in the cytoplasm (FIGs. 5G and 5K). However, when first incubated (pre-incubated) with 1.8 µM of plasma gelsolin, LPS had no discernable effect on the actin cytoskeletons of either the endothelial cells or the astrocytes. The addition of gelsolin completely blocked the LPS-induced translocation of NF-κB (FIGs. 5G-4N), as shown by immunostaining for NF-kB with an antibody against NF-kB (FIGs. 5G-5J), and cell nuclei with DAPI (FIGs. 5L-5M). Preincubation with 1.8 µM gelsolin blocked LPS-induced translocation (FIGs. 5J and 4N).

### Protective effect of QRLFQVKGRR on LPS-mediated alteration of platelet function -

To isolate human platelets, blood from healthy volunteers was collected in acid-citrate dextrose. Platelet-rich plasma obtained after centrifugation (15 minutes, 110 x g) at room temperature, was supplemented with apyrase (0.5U/ml) (Sigma-Aldrich) to degrade excess nucleotides. Platelets were sedimented by centrifugation (10 minutes, 1000 x g), suspended in buffer A (139 mM NaCl, 2.8 mM KCl, 0.8mM MgCl₂, 0.8 mM KH₂PO₄, 8.9 mM NaHCO₃, 10 mM HEPES, 5.6 mM glucose, 0.3 % albumin, pH 7.35) and filtered on a 50 ml column of Sepharose 3B to obtain gel-filtered platelets (GFP).

Platelet aggregation was initiated by adding collagen (10 µg/ml) (Chrono-Log Corp., Havertown, PA). Platelet aggregation was monitored using a Chronolog Lumi-Aggregometer (Chrono-Log Corp.). Changes in light transmission were recorded for 10 minutes, using a PowerLab/200 instrument and MacLab Chart program Version 3 .2. When required before activation, the platelet suspension was reacted with LPS, or LPS-preincubated with the SEQID No:1 peptide (QRLFQVKGRR).

A shown in FIG. 6, LPS aggregation was stimulated by collagen, but aggregation was inhibited and reversed by the SEQID No:1 peptide (QRLFQVKGRR). When added alone, LPS strongly inhibited the aggregation and secretion of platelets induced by collagen. Addition of gelsolin SEQID No:1 to LPS before its addition to platelets restored, in a peptide concentration-dependent manner, the normal collagen-induced platelets activation. Gelsolin SEQID No:1 by itself had no effect on collagen-mediated platelet functions.

### Example 5 - Effect In Vivo of Plasma Gelsolin on Administered LPS

Since humans with sepsis have decreased plasma gelsolin concentrations, the effect of LPS administration on gelsolin levels was examined in mice. 6-7 week old, male, wild type C57BL/6 mice, each weighing 18-20 grams, purchased from Charles River Laboratories, Wilmington, MA, were used for all animal studies. Mice were given free access to a standard feed and water, and all procedures and studies described here have been approved by Harvard Medical Area Standing Committee on Animals according to standards as set forth in The Guide for the Care and Use of Laboratory Animals.

In a dose response assay, animals were injected i.p. (intraperitoneally) with LPS (P. *aeruginosa* Serotype 10, Sigma) at doses of 0, 10, 20, and 40mg/kg. 3-4 animals were used in each group. 24 hours after administration of the LPS, the animals were anesthetized with Avertin i.p. (Fluka Chemie, Switzerland) at dose of 0.015-0.017mg/g. Blood was then collected by retro-orbital bleeding into 0.1 volume of Aster-Jandl anticoagulant and centrifuged at 1000 x g for 10 minutes. Plasma was aspirated and frozen in liquid nitrogen and stored in -80°C until analyzed.

After sacrificing the anesthetized animals by cervical dislocation, peritoneal lavage was performed by injecting 3 ml of sterile PBS into the mouse peritoneal cavity. After gently messaging the abdomen, the fluid was aspirated by a syringe connected to a 22-gauge needle and centrifuged at 1000 x g for 10 minutes to remove cellular contents. The supernatant fluid was collected and frozen in liquid nitrogen and stored at -80°C until analyzed.

To study the effect on mortality, animals were injected i.p. with 25mg/kg LPS *(E. coli* 055:B5, Sigma) and each mouse was randomly selected to receive one of the following three treatments: 1) GSN (containing: 8 mg of recombinant human plasma gelsolin (Biogen-IDEC, Inc) containing 1 mM Ca was dissolved in 400 µl of 0.15 M NaCl (saline); or 2) BSA (containing: 8 mg of bovine albumin (Serologicals Proteins, Inc, Kankakee, IL) containing 1 mM Ca dissolved in 400 µl of saline, or 3) saline (containing only 400 µl saline). The GSN. (gelsolin), BSA or saline solutions were administered subcutaneously at the dorsal surface of each animal immediately after LPS instillation, and then repeated at 24, 48, and 72 hours. There were 9 mice in the saline group and 8 mice each in the GSN group and the BSA group. Animals were monitored frequently, and mortality was recorded and presented at an interval of days. Animal mortality study data are presented as Kaplan-Meier curves and log-rank test was used to analyze treatment impact on animal mortality. A *p*-value less than 0.05 was considered significant.

To measure gelsolin *in vivo,* a nucleation assay for plasma gelsolin was performed as follows: the collected mouse plasma was diluted 1:5 in Buffer B (0.1 M KCl, 0.2mM MgCl₂, 1 mM EGTA (ethyleneglycol-bis(2-aminoethyl) N,N,N',N',-tetraacetic acid), 0.5 mM ATP, 0.5 mM β-mercaptoethanol, 10 mM Tris-HCl buffer, pH 7.4). From the diluted plasma sample, 5 µl was added to 280 µl Buffer B supplemented with 1.5 mm CaCl₄ and 0.4 µM phallacidin in 6 x 50 mm borosilicate culture tubes. The actin polymerization reaction was initiated by adding 15 µl 20 µM pyrene-actin in Buffer A (0.5 mM ATP, 0.5 mM β-mercaptoethanol, 0.2 mM CaCl₂ 0.2 mM Tris-HCl buffer, pH 7.4). Polymerization was monitored for 200 seconds in a spectrofluorimeter at excitation and emission wavelengths of 366 and 386 nm respectively. Gelsolin concentrations were estimated from a standard curve using recombinant human plasma gelsolin.

For all of fluorescence measurements in this or the foregoing Examples or in future assays, initial studies will be done with a standard fluorimeter, for which a sample holder is adapted to preferably permit measurements from samples as small as 200 µL in disposable, pyrogen-free glass tubes. The pyrogen-free aspect is irrelevant for most studies since relatively larger amounts (ng/ml to µg/ml) of LPS will be added. But for other studies using specialized reagents, use of standard fluorimeter cuvettes is impractical since minimization of sample volume is needed to reduce the cost of the specialized reagents. The fluorescence intensity of PBP10 is high enough to read at 2 µM levels, and the fluorescence intensity will only go up when binding LPS.

Measurement of peritoneal lavage gelsolin was performed similarly, except 50 µl of peritoneal lavage and 30 µl of pyrene actin were used instead. Stock pyrene actin for these assays, prepared by the method of Kouyama and Mihashi (Kouyama et al., Eur. J. Biochem. 114:33-38 (1981), was stored at -80°C in lots, thawed and diluted 10x with Buffer A, centrifuged at 250k x g for 30 minutes after standing overnight.

Plasma gelsolin was measured by the actin nucleation assay described above, and repeated (each assay was done in duplicate). All values are presented as mean ±SD. A nonparametric test, Spearman Rank Correlation was used to analyze correlations in the dose response study. Mann-Whiney U-test was performed when comparing gelsolin levels. Plasma albumin levels were measured colorimetrically using a commercial kit (Stanbio Laboratory, Boerne, TX) according to the manufacture's instruction.

As shown in FIG. 7, when mice receiving lethal dose of LPS intraperitoneally were treated with gelsolin (GSN), albumin (BSA) or saline, respectively, the exogenous plasma gelsolin rescued the endotoxemic mice. This was a dramatic response to gelsolin treatment, since mice receiving either the BSA or saline therapies, all died within 6 days of LPS treatment. See FIG. 7. By comparison, at Day 7, the surviving mice (all in the GSN group) all appeared to have completely recovered, and exhibited no signs of distress. The gelsolin amounts administered by this route restored plasma levels in LPS-treated animals to 200 µg/ml, which is slightly greater than the baseline concentrations (data not shown). Therefore, it can be concluded that animals treated *in vivo* with plasma gelsolin have markedly improved survival compared to those treated with saline or BSA controls (FIG. 7) (p< 0.001).

### Example 6 -Function of Plasma Gelsolin in Vivo in Sepsis

*Plasma gelsolin as a treatment for sepsis* - In light of the foregoing data in Example 5, suggesting that administration of exogenous plasma gelsolin can improve survival in endotoxemic animals, gelsolin-null and wild-type mice are exposed to similar endotoxemic challenge and determine if there are differences in survival and in markers of inflammation. Moreover, although it is not yet definitely known through which inflammation pathway, inflammation plasma gelsolin exerts its effect. Consequently, exogenous plasma gelsolin administration improves survival of wild-type septic animals, decreases secondary organ damage, and is associated with lower inflammatory mediators.

The general design of the experiment employs two different models of sepsis: 1) the LPS induced endotoxemia model, and 2) the cecal ligation puncture (CLP) mouse model. The main reason to develop the CLP model is that it more closely resembles clinical sepsis than LPS (Schultz et al., Ann. Aled. 34:573-581 (2002)). In practice, gelsolin-null and genetically similar wild-type (C57BL/6) mice are subjected to injury (LPS or CLP). Two hours after injury, wild-type animals are divided randomly into 3 groups: 1) an experimental group given plasma gelsolin subcutaneously and 2 control groups: 2) a group given PBS; and 3) a control group given only BSA protein. Six animals from each group are then anesthetized and bled at various time points (4 hr, 16 hr, 28 hr, 40 hr), from which plasma will be generated.

The plasma samples are then analyzed for plasma gelsolin levels, as well as for inflammatory factors, TNF-α, IL-1β, and IL-6, since these mediators are known to increase during the development of sepsis (see, *e.g.,* Riedemann et al., J. Clin. Invest. 112:460-467 (2003)). Furthermore, high mobility group B-1 (HMGB-1) protein is also measured, since this protein has been correlated with the delayed death of septic animals (Wang et al., Science 285:248-251 (1999)). To measure the secondary organ damage, plasma transaminases (ALT, AST), LDH, and creatinine are measured and compared. Additionally, there will be 10 animals in each arm of the study that are subjected to CLP in the "death as end point" study. Based on the data above, this number of animals is sufficient to most likely yield statistically significant differences in mortality between control and experimental animals.

Based upon the previous data, at least from Example 5 with LPS septic mice, gelsolin-null animals should have significantly higher mortality, higher inflammatory markers, and more severe secondary organ damage than the wild-type animals. Furthermore, plasma gelsolin treatment will significantly lower the mortality rate of wild-type animals subjected to CLP. Moreover, it is anticipated that gelsolin-treated animals will have less organ dysfunction reflected by lower levels of transaminases, LDH and creatinine. Results from CLP mice, therefore, lend more support to gelsolin as a promising therapeutic treatment in clinical sepsis.

The cytokine profile differs somewhat between LPS and CLP models of sepsis, especially in TNF-α production (Villa et al., Clin. Diagn. Lab. Immunol. 2:549-553 (1995)). In the LPS model, TNF-α is intensely elevated within 2 hours of challenge (see Rothenbach *et al.,* 2004, *supra.*)*;* whereas in the CLP model, TNF-α is only mildly elevated. Since the present animals are treated 2 hours after injury, it is likely that TNF-α pathway has already been initiated, and gelsolin treatment is unlikely to affect TNF-α levels. On the other hand IL-1β and IL-6 are elevated in both the LPS and CLP models, and tend to stay elevated for many hours, meaning that gelsolin treatment may affect these 2 cytokines in reducing inflammation. HMGB-1 appears later in the course of sepsis, and is associated with lethality (Wang *et al.,* 1999, *supra).* As a result, gelsolin treatment may decrease blood HMGB-1 levels, reflecting the rescue effect of the gelsolin treatment.

Given that a preliminary study on trauma patients shows that there is a significant difference in the plasma gelsolin levels between surviving and non-surviving critically ill patients, the study is extended to include plasma gelsolin levels in patients with sepsis, burns and ARDS. In general, discarded anti-coagulated blood is collected that has been drawn from the patients. Plasma is generated from each sample and flash-frozen in liquid nitrogen and stored in -80° C until measurements of plasma gelsolin and albumin levels are performed. For each patient, the follow data are recorded: APACHE II scores, ventilated time, ICU days, hospital days, age, gender, race, development of ARDS and sepsis, ICU mortality and hospital mortality. The primary end points of the study include ICU mortality and hospital mortality. Secondary end points include 30-day ICU free days, 30-day ventilator-free hours, development of ARDS, and sepsis.

By assuming alpha = 0.05 (thereby establishing a p value of 0.05), the estimated incidence of ARDS and sepsis will equal about 15%; which determines the sample size needed to generate an 80% power of a two-sided Fisher's exact test being able to detect an OR of 10.28 (which is the estimated relative risk of ARDS or sepsis of patients with very low gelsolin, compared to low to normal gelsolin levels) between very low plasma gelsolin and development of ARDS or sepsis is 67 patients. It is expected that there will be a significant difference in plasma gelsolin levels between survivors and non-survivors in the MICU, as supported by above studies. Moreover, it is further expect that those with very low plasma gelsolin levels will have significantly shorter ICU-free days, ventilator-free hours and will be at higher risk of developing ARDS and/or sepsis. Albumin level serves as the control protein to show that depression of plasma gelsolin is not simply representative of a non-specific low protein state, which conclusion is also supported by the data above.

## Claims

1. Gelsolin, or functionally equivalent peptide fragment thereof, for use as a medicament in a method of preventing, neutralizing or reducing endotoxemia or endotoxin-induced septic shock in a patient in *vivo,* wherein the patient is subject to or susceptible to gram negative bacterial infection.

2. Gelsolin for use as claimed in claim 1, wherein the gelsolin, or functionally equivalent peptide fragment thereof, is for parenteral administration.

3. Gelsolin for use as claimed in claim 2, wherein following administration of the therapeutically effective amount of gelsolin, or functionally equivalent peptide fragment thereof, the patient's blood or extracellular fluid comprises an increased concentration of gelsolin, as compared with the level of gelsolin before administration.

4. Gelsolin for use as claimed in either claim 2 or 3, wherein the increased concentration of gelsolin in the patient protects the patient from endotoxemia or endotoxin-induced sepsis.

5. Gelsolin for use as claimed in any of claims 2 to 4, wherein the increased concentration of gelsolin in the patient decreases the concentration of bacterial LPS in the patient.

6. Gelsolin for use as claimed in any of claims 2 to 5, wherein endotoxemia or endotoxin-induced sepsis in the patient is LPS-induced following the bacterial infection or triggering of endotoxins in the patient.

7. Gelsolin for use as claimed in either claim 2 or 3, wherein the increased concentration of gelsolin in the patient decreases, ameliorates or prevents bacterial LPS-induced disruption of the patient's cellular responses or formation of toxic structures.

8. Gelsolin for use as claimed in any one of claims 1 to 7, the method further comprises enhancing the endogenous LBP (LPS binding protein) activity, thereby inhibiting, ameliorating, or preventing incorporation of LPS into blood lipoproteins of the patient.

9. Gelsolin for use as claimed in any one of claims 1 to 8, wherein the gelsolin, or functionally equivalent peptide fragment thereof, comprises plasma gelsolin.

10. Gelsolin for use as claimed in any one of claims 1 to 9, wherein the gelsolin, or functionally equivalent peptide fragment thereof, comprises amino acid residues 160-169 of gelsolin.

11. Gelsolin for use as claimed in any one of claims 1 to 10, wherein the gelsolin, or functionally equivalent peptide fragment thereof, comprises recombinantly produced or expressed plasma gelsolin.

12. Gelsolin for use as claimed in any one of claims 1 to 11, wherein the gelsolin, or functionally equivalent peptide fragment thereof, comprises SEQID No: 1.

13. A pharmaceutical composition comprising gelsolin for use as claimed in any of claims 1 to 12, said pharmaceutical composition comprising:
(1) gelsolin, or functionally equivalent peptide fragment thereof, wherein such gelsolin or said fragment is substantially free of natural contaminants; and
(2) a pharmaceutically acceptable vehicle.

14. The pharmaceutical composition for use as claimed in claim 13, wherein the pharmaceutical composition further comprises a sufficient amount of Ca²⁺ to activate the binding capability of exogenously administered gelsolin.

15. A pharmaceutical composition comprising:
(1) gelsolin, or functionally equivalent peptide fragment thereof, wherein such gelsolin or said fragment is substantially free of natural contaminants; and
(2) a pharmaceutically acceptable vehicle; and
(3) a sufficient amount of Ca²⁺ to activate the binding capability of exogenously administered gelsolin,
for use in a method of preventing, neutralizing or reducing endotoxemia or endotoxin-induced septic shock in a patient.

16. The use of gelsolin, or functionally equivalent peptide fragment thereof, in the manufacture of a medicament for preventing, neutralizing or reducing endotoxemia or endotoxin-induced septic shock in a patient.

17. Gelsolin for use in a method of predicting clinical outcome associated with massive inflammation in a patient susceptible to inflammatory shock or endotoxin-induced sepsis, said method comprising measuring the circulating gelsolin concentration in the patient, wherein a decrease ≤30% of normal pre-trauma or pre-infection gelsolin levels predicts such adverse outcome and predicts a need for gelsolin therapy.

18. Gelsolin for use as claimed in claim 17, comprising the use of fluorescent phosphorylated inositol phospholipid derivatives or tritium labeling as a method for detecting an endotoxin in an *in vitro* sample.

## Patentansprüche

1. Gelsolin, oder funktionell äquivalentes Peptidfragment davon, zur Verwendung als Medikament bei einem Verfahren zum Präventieren, Neutralisieren oder Reduzieren von Endotoxämie oder endotoxininduziertem septischem Schock bei einem Patienten *in vivo,* wobei der Patient einer gramnegativen bakteriellen Infektion ausgesetzt oder für diese anfällig ist.

2. Gelsolin zur Verwendung gemäß Anspruch 1, wobei das Gelsolin, oder funktionell äquivalente Peptidfragment davon, für eine parenterale Verabreichung gedacht ist.

3. Gelsolin zur Verwendung gemäß Anspruch 2, wobei nach der Verabreichung der therapeutisch wirksamen Menge von Gelsolin, oder des funktionell äquivalenten Peptidfragments davon, das Blut oder die extrazelluläre Flüssigkeit des Patienten im Vergleich zu dem Niveau von Gelsolin vor der Verabreichung eine erhöhte Konzentration von Gelsolin umfasst.

4. Gelsolin zur Verwendung gemäß Anspruch 2 oder 3, wobei die erhöhte Konzentration von Gelsolin bei dem Patienten den Patienten vor Endotoxämie oder endotoxininduzierten septischen Schock schützt.

5. Gelsolin zur Verwendung gemäß einem der Ansprüche 2 bis 4, wobei die erhöhte Konzentration von Gelsolin bei dem Patienten die Konzentration von bakteriellen LPS bei dem Patienten verringert.

6. Gelsolin zur Verwendung gemäß einem der Ansprüche 2 bis 5, wobei Endotoxämie oder endotoxininduzierter septischer Schock bei dem Patienten nach der bakteriellen Infektion oder Auslösen von Endotoxinen LPS-induziert wird.

7. Gelsolin zur Verwendung gemäß Anspruch 2 oder 3, wobei die erhöhte Konzentration von Gelsolin bei dem Patienten eine bakterielle LPS-induzierte Störung der zellulären Antworten des Patienten oder eine Bildung von toxischen Strukturen verringert, abschwächt oder präventiert.

8. Gelsolin zur Verwendung gemäß einem der Ansprüche 1 bis 7, wobei das Verfahren ferner eine Verstärkung der endogenen LBP-Aktivität (LBP = LPS-bindendes Protein) umfasst, wodurch eine Einbindung von LPS in Blut-Lipoproteine des Patienten gehemmt, abgeschwächt oder präventiert wird.

9. Gelsolin zur Verwendung gemäß einem der Ansprüche 1 bis 8, wobei das Gelsolin, oder funktionell äquivalente Peptidfragment davon, Plasma-Gelsolin umfasst.

10. Gelsolin zur Verwendung gemäß einem der Ansprüche 1 bis 9, wobei das Gelsolin, oder funktionell äquivalente Peptidfragment davon, Aminosäurereste 160-169 von Gelsolin umfasst.

11. Gelsolin zur Verwendung gemäß einem der Ansprüche 1 bis 10, wobei das Gelsolin, oder funktionell äquivalente Peptidfragment davon, rekombinant produziertes oder exprimiertes Plasma-Gelsolin umfasst.

12. Gelsolin zur Verwendung gemäß einem der Ansprüche 1 bis 11, wobei das Gelsolin, oder funktionell äquivalente Peptidfragment davon, SEQ-ID-Nr. 1 umfasst.

13. Pharmazeutische Zusammensetzung, umfassend Gelsolin zur Verwendung gemäß einem der Ansprüche 1 bis 12, wobei die pharmazeutische Zusammensetzung Folgendes umfasst:
(1) Gelsolin, oder funktionell äquivalentes Peptidfragment davon, wobei ein solches Gelsolin oder das genannte Fragment im Wesentlichen frei von natürlichen Kontaminanten ist; und
(2) einen pharmazeutisch akzeptablen Hilfsstoff.

14. Pharmazeutische Zusammensetzung gemäß Anspruch 13, wobei die pharmazeutische Zusammensetzung ferner eine ausreichende Menge von Ca²⁺ umfasst, um die Bindefähigkeit von exogen verabreichtem Gelsolin zu aktivieren.

15. Pharmazeutische Zusammensetzung, umfassend:
(1) Gelsolin, oder funktionell äquivalentes Peptidfragment davon, wobei ein solches Gelsolin oder das genannte Fragment im Wesentlichen frei von natürlichen Kontaminanten ist; und
(2) einen pharmazeutisch akzeptablen Hilfsstoff; und
(3) eine ausreichende Menge von Ca²⁺, um die Bindefähigkeit von exogen verabreichtem Gelsolin zu aktivieren,
zur Verwendung bei einem Verfahren zum Präventieren, Neutralisieren oder Reduzieren von Endotoxämie oder endotoxininduziertem septischem Schock bei einem Patienten.

16. Verwendung von Gelsolin, oder des funktionell äquivalenten Peptidfragments davon, bei der Herstellung eines Medikaments zum Präventieren, Neutralisieren oder Reduzieren von Endotoxämie oder endotoxininduziertem septischem Schock bei einem Patienten.

17. Gelsolin zur Verwendung bei einem Verfahren zum Prognostizieren eines klinischen Ergebnisses, das mit einer massiven Entzündung bei einem Patienten assoziiert ist, der für Entzündungsschock oder endotoxininduzierte Sepsis anfällig ist, wobei das genannte Verfahren das Messen der Konzentration von zirkulierendem Gelsolin bei dem Patienten umfasst, wobei eine Verringerung von ≤30 % der normalen Prä-Trauma- oder Prä-Infektions-Gelsolin-Niveaus ein solches nachteiliges Ergebnis prognostiziert und eine Notwendigkeit für Gelsolin-Therapie prognostiziert.

18. Gelsolin zur Verwendung gemäß Anspruch 17, das die Verwendung von fluoreszierenden, phosphorylierten Inositol-Phospholipid-Derivaten oder Tritium-Markierung als Verfahren zum Detektieren eines Endotoxins in einer *In-vitro-Probe* umfasst.

## Revendications

1. Gelsoline, ou fragment peptidique fonctionnellement équivalent de celle-ci, pour une utilisation comme médicament dans une méthode de prévention, neutralisation ou réduction de l'endotoxémie ou du choc septique induit par des endotoxines chez un patient *in vivo,* le patient étant sujet à ou à risque d'infection par des bactéries gram négatives.

2. Gelsoline pour une utilisation telle que revendiquée dans la revendication 1, la gelsoline ou le fragment peptidique fonctionnellement équivalent de celle-ci étant administré par voie parentérale.

3. Gelsoline pour une utilisation telle que revendiquée dans la revendication 2, le sang ou le liquide extracellulaire du patient présentant après l'administration de la quantité thérapeutiquement efficace de gelsoline ou du fragment peptidique fonctionnellement équivalent de celle-ci, une augmentation de la concentration de gelsoline, par rapport au niveau de gelsoline avant l'administration.

4. Gelsoline pour une utilisation telle que revendiquée dans la revendication 2 ou 3, l'augmentation de la concentration de gelsoline chez le patient protégeant le patient de l'endotoxémie ou du sepsis induit par des endotoxines.

5. Gelsoline pour une utilisation telle que revendiquée dans l'une quelconque des revendications 2 à 4, l'augmentation de la concentration de gelsoline chez le patient diminuant la concentration de LPS bactériens chez le patient.

6. Gelsoline pour une utilisation telle que revendiquée dans l'une quelconque des revendications 2 à 5, l'endotoxémie ou le sepsis induits par des endotoxines chez le patient étant induits par les LPS suite à l'infection bactérienne ou à l'activation d'endotoxines chez le patient.

7. Gelsoline pour une utilisation telle que revendiquée dans la revendication 2 ou 3, l'augmentation de la concentration de gelsoline chez le patient diminuant, améliorant ou prévenant la perturbation des réponses cellulaires ou la formation de structures toxiques induites par les LPS bactériens chez le patient.

8. Gelsoline pour une utilisation telle que revendiquée dans l'une quelconque des revendications 1 à 7, la méthode comprenant en outre l'augmentation de l'activité de la LBP (protéine liant les LPS) endogène, inhibant, améliorant ou prévenant ainsi l'incorporation des LPS dans les lipoprotéines sanguines du patient.

9. Gelsoline pour une utilisation telle que revendiquée dans l'une quelconque des revendications 1 à 8, la gelsoline, ou le fragment peptidique fonctionnellement équivalent de celle-ci, comprenant la gelsoline plasmatique.

10. Gelsoline pour une utilisation telle que revendiquée dans l'une quelconque des revendications 1 à 9, la gelsoline, ou le fragment peptidique fonctionnellement équivalent de celle-ci, comprenant les résidus acides aminés 160 à 169 de la gelsoline.

11. Gelsoline pour une utilisation telle que revendiquée dans l'une quelconque des revendications 1 à 10, la gelsoline ou le fragment peptidique fonctionnellement équivalent de celle-ci comprenant la gelsoline plasmatique produite ou exprimée par voie recombinante.

12. Gelsoline pour une utilisation telle que revendiquée dans l'une quelconque des revendications 1 à 11, la gelsoline, ou le fragment peptidique fonctionnellement équivalent de celle-ci, comprenant SEQID No : 1.

13. Composition pharmaceutique comprenant de la gelsoline pour une utilisation telle que revendiquée dans une quelconque des revendications 1 à 12, ladite composition pharmaceutique comprenant :
(1) de la gelsoline, ou un fragment peptidique fonctionnellement équivalent de celle-ci, ladite gelsoline ou ledit fragment étant sensiblement exempts de contaminants naturels ; et
(2) un véhicule pharmaceutiquement acceptable.

14. Composition pharmaceutique pour une utilisation telle que revendiquée dans la revendication 13, la composition pharmaceutique comprenant en outre une quantité suffisante de Ca²⁺ pour activer la capacité de liaison de la gelsoline administrée de manière exogène.

15. Composition pharmaceutique comprenant :
(1) de la gelsoline, ou un fragment peptidique fonctionnellement équivalent de celle-ci, ladite gelsoline ou ledit fragment étant sensiblement exempts de contaminants naturels ; et
(2) un véhicule pharmaceutiquement acceptable ; et
(3) une quantité suffisante de Ca²⁺ pour activer la capacité de liaison de la gelsoline administrée de manière exogène,
pour une utilisation dans une méthode de prévention, neutralisation ou réduction de l'endotoxémie ou du choc septique induit par des endotoxines chez un patient.

16. Utilisation de la gelsoline, ou d'un fragment peptidique fonctionnellement équivalent de celle-ci, dans la fabrication d'un médicament pour la prévention, la neutralisation ou la réduction de l'endotoxémie ou du choc septique induit par une endotoxine chez un patient.

17. Gelsoline pour une utilisation dans une méthode de prédiction d'un résultat clinique associé à une inflammation massive chez un patient à risque de choc inflammatoire ou de sepsis induit par une endotoxine, ladite méthode comprenant le dosage de la concentration de gelsoline circulante chez le patient, une diminution ≤ 30 % des taux de gelsoline normaux pré-traumatisme ou pré-infection prédisant un tel résultat défavorable et prédisant un besoin en traitement par la gelsoline.

18. Gelsoline pour une utilisation telle que revendiquée dans la revendication 17, comprenant l'utilisation de dérivés phospholipidiques inositol phosphorylés fluorescents ou de marquage au tritium comme méthode de détection d'une endotoxine dans un échantillon *in vitro.*
